# EUROPEAN PATENT APPLICATION

(11) **EP 4 711 440 A1**
(43) Date of publication of application: **18.03.2026**
(21) Application number: 24803425.8
(22) Date of filing: 30.04.2024
(51) Int. Cl.: C12M 1/34, C12N 15/10, C12Q 1/04, C12Q 1/6844

(54) **DETECTION DEVICE, DETECTION METHOD, AND HEATING DEVICE**

(30) Priority: 08.05.2023 JP 2023076782
(71) Applicant: National Institute of Advanced Industrial Science and Technology, Chiyoda-ku Tokyo 100-8921 (JP); Teikyo University, Tokyo 173-8605 (JP); Keio University, Tokyo, 108-8345 (JP)
(72) Inventor: MIYAMURA, Hiroyuki, Tsukuba-shi, Ibaraki 305-8560 (JP); SUZUKI, Koichi, Tokyo 173-8605 (JP); MIKITA, Kei, Tokyo 160-8582 (JP)
(74) Representative: Mathys & Squire
(86) International application number: PCT/JP2024/016682
(87) International publication number: WO 2024/232313

(57) **Abstract**

Provided are a detection apparatus capable of detecting a nucleic acid without rely on the user's expertise or skill, a detection method using the detection apparatus, and a heating apparatus for heating the detection apparatus. The detection apparatus is a detection apparatus (1) that detects an amplified nucleic acid generated from a target nucleic acid contained in a specimen and the detection apparatus includes a specimen placement member (22) in which the specimen is placed, a test strip (29) used for detecting the amplified nucleic acid, and an amplification member (50, *51,* 52, 53) on which the reagent for use in generating the amplified nucleic acid from the target nucleic acid is immobilized, and a supply line (19, 25, 26, 27, 28) that supplies the amplified nucleic acid toward the test strip, in which the amplification member is disposed in the supply line.

## Description

### [Technical Field]

The present invention relates to a detection apparatus, a detection method, and a heating apparatus.

### [Background Art]

The genetic testing method is a method for readily determining, for example, whether or not a subject is infected with a pathogen, using a nucleic acid such as a gene (DNA or RNA).

The genetic testing method detects a nucleic acid (hereinafter referred to as "amplified nucleic acid") that is generated by amplifying a nucleic acid (hereinafter referred to as "target nucleic acid") derived from a microorganism (e.g., bacterium, fungus, virus) contained in a specimen (e.g., saliva of a subject) collected from the subject.

A user who performs the genetic testing method (e.g., a medical professional) performs a step of amplifying the target nucleic acid (hereinafter referred to as "amplification step") and a step of detecting the amplified nucleic acid (hereinafter referred to as "detection step"). The user performs pipetting to mix a specimen and reagents in each of the amplification step and the detection step.

The user is required to have specialized knowledge and techniques regarding mixing of specimens and reagents. That is, the accuracy of testing by the genetic testing method depends on the user's expertise and skill.

Detection apparatuses for detecting whether or not a detection target is contained in a specimen have been proposed (e.g., see JP 2022-070662 A).

### [Citation List]

### [Patent Literature]

[PTL 1] JP 2022-070662 A

### [Summary of Invention]

### [Technical Problem]

The present invention is directed to providing a detection apparatus capable of detecting a nucleic acid without relying on the user's expertise or skill, a detection method using the detection apparatus, and a heating apparatus for heating the detection apparatus.

### [Solution to Problem]

A detection apparatus according to an aspect of the present invention is a detection apparatus that detects an amplified nucleic acid generated from a target nucleic acid contained in a specimen and the detection apparatus includes a specimen placement member in which the specimen is placed, a test strip used for detecting the amplified nucleic acid, an amplification member on which a reagent for use in generating the amplified nucleic acid from the target nucleic acid is immobilized, and a supply line that supplies the amplified nucleic acid toward the test strip, in which the amplification member is disposed in the supply line.

### [Advantageous Effects of Invention]

The present invention is capable of providing the detection apparatus capable of detecting a nucleic acid without relying on the user's expertise or skill, a detection method using the detection apparatus, and a heating apparatus for heating the detection apparatus.

### [Brief Description of Drawings]

[Fig. 1] Fig. 1 is a schematic diagram illustrating a relationship among a specimen, a reagent, and a developing solution that are used in a detection apparatus according to one or more embodiments of the present invention.
[Fig. 2] Fig. 2 is a perspective view of the detection apparatus illustrating an embodiment of the detection apparatus.
[Fig. 3] Fig. 3 is an exploded perspective view of the detection apparatus in Fig. 2.
[Fig. 4] Fig. 4 is a schematic diagram illustrating a state in which each amplification member is extended through each first supply line of the detection apparatus in Fig. 2.
[Fig. 5] Fig. 5 is a schematic diagram illustrating a state before a heater is attached to the detection apparatus in Fig. 2.
[Fig. 6] Fig. 6 is a schematic diagram illustrating a state after the heater is attached to the detection apparatus in Fig. 2.
[Fig. 7] Fig. 7 is an exploded perspective view of the detection apparatus illustrating another embodiment of the detection apparatus.
[Fig. 8] Fig. 8 is a schematic diagram illustrating a state in which a second button of the detection apparatus in Fig. 2 or Fig. 7 is being operated.
[Fig. 9] Fig. 9 is a schematic diagram illustrating a state in which an amplification member of the detection apparatus in Fig. 2 or Fig. 7 is connected to a chromatography paper of the detection apparatus.
[Fig. 10] Fig. 10 is a schematic diagram illustrating another embodiment of the detection apparatus according to the present invention, illustrating a state in which the second button of the detection apparatus is being operated.
[Fig. 11] Fig. 11 is a schematic diagram illustrating a state in which the amplification member of the detection apparatus in Fig. 10 is connected to the chromatography paper of the detection apparatus.
[Fig. 12] Fig. 12 is a schematic diagram illustrating yet another embodiment of the detection apparatus according to the present invention, illustrating a state in which the second button of the detection apparatus is being operated.
[Fig. 13] Fig. 13 is a schematic diagram illustrating a state in which the amplification member of the detection apparatus in Fig. 12 is connected to the chromatography paper of the detection apparatus.
[Fig. 14] Fig. 14 is a schematic diagram illustrating yet another embodiment of the detection apparatus according to the present invention, illustrating a state in which the second button of the detection apparatus is being operated.
[Fig. 15] Fig. 15 is a schematic diagram illustrating a state in which the amplification member of the detection apparatus in Fig. 14 is connected to the chromatography paper of the detection apparatus.
[Fig. 16] Fig. 16 is a schematic diagram illustrating an embodiment of a heating apparatus according to the present invention.
[Fig. 17] Fig. 17 is a schematic diagram illustrating a state, as viewed from a motor side of the heating apparatus in Fig. 16, in which a first button pressing portion, a second button pressing portion, and a motor rotary shaft of the heating apparatus are rotating.
[Fig. 18] Fig. 18 is a schematic diagram illustrating a state in which a first button and the second button of the detection apparatus in Fig. 7 are pressed by a first button pressing portion and a second button pressing portion of the heating apparatus in Fig. 16, respectively.

### [Description of Embodiments]

Embodiments of a detection apparatus (hereinafter referred to as "present apparatus"), a detection method (hereinafter referred to as "present method"), and a heating apparatus (hereinafter referred to as a present heating apparatus) according to the present invention will now be described with reference to the drawings.

The following description is an example of when the present apparatus is used to determine whether a subject is infected with a specific pathogen (e.g., influenza virus). When a nucleic acid derived from a specific pathogen is contained in a specimen collected from a subject, the present apparatus detects, by using chromatography paper, an amplified nucleic acid generated by amplification of the nucleic acid. The chromatography paper develops color when the amplified nucleic acid is detected. A user of the present apparatus (e.g., a medical professional or a subject) determines whether the subject is infected with the specific pathogen, based on whether the chromatography paper has developed color.

Herein, the nucleic acid derived from the specific pathogen is an example of a target nucleic acid in the present invention. Saliva to be collected from a subject is an example of a specimen in the present invention. The chromatography paper is an example of a test strip in the present invention.

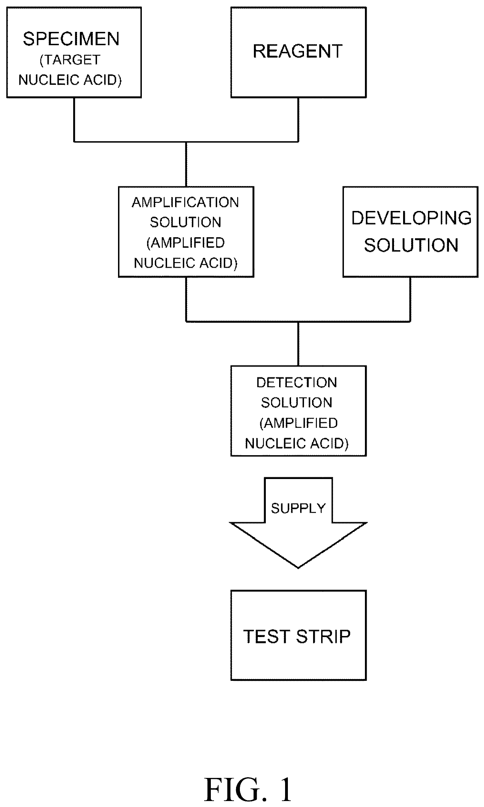

Fig. 1 is a schematic diagram illustrating a relationship among a specimen, a reagent, and a developing solution that are used in the present apparatus. The figure illustrates an example of a case in which a subject is infected with the specific pathogen.

The figure illustrates that the target nucleic acid is contained in the specimen. The figure illustrates that the specimen is mixed with the reagent. The figure illustrates that an amplification solution is generated as a result of the mixing. The figure illustrates that the amplification solution contains the amplified nucleic acid. The figure illustrates that the amplification solution is mixed with the developing solution. The figure illustrates that a detection solution is generated as a result of the mixing. The figure illustrates that the detection solution contains the amplified nucleic acid. The figure illustrates that the detection solution is supplied to the test strip.

Saliva is a specimen that is collected from a subject to determine whether the subject is infected with a specific pathogen. Saliva contains a target nucleic acid when the subject is infected with the specific pathogen. Saliva does not contain the target nucleic acid when the subject is not infected with the specific pathogen. The saliva for use in the present apparatus may be saliva itself, or saliva diluted with physiological saline or the like containing an RNA-degrading enzyme inhibitor or a denaturant.

The reagent is a reagent for use in amplifying a target nucleic acid. The method for amplifying a target nucleic acid in the present invention is an isothermal nucleic acid amplification method. The method for amplifying the target nucleic acid in the present embodiment is a loop-mediated isothermal amplification (LAMP) method, which is a type of isothermal nucleic acid amplification method. The reagent in the present embodiment is a LAMP reagent.

The amplification solution is a solution generated by the reaction between a specimen and a reagent. The amplification solution contains an amplified nucleic acid when the specimen contains a target nucleic acid. The amplification solution does not contain the amplified nucleic acid when the specimen does not contain the target nucleic acid.

The developing solution is a medium (a solution) for moving the amplified nucleic acid toward the chromatography paper. The developing solution in the present embodiment is a mixed solution of water, a phosphate buffer, sodium chloride, a surfactant such as Tween20, and bovine serum albumin.

The detection solution is a solution to be supplied to the chromatography paper. The detection solution is generated by mixing the amplification solution with the developing solution. The detection solution contains the amplified nucleic acid when the amplification solution contains the amplified nucleic acid. The detection solution does not contain the amplified nucleic acid when the amplification solution does not contain the amplified nucleic acid.

The target nucleic acid is a nucleic acid derived from a specific pathogen that may be contained in a specimen. The target nucleic acid is a nucleic acid to be subjected to amplification by the present apparatus.

The amplified nucleic acid is a target nucleic acid amplified by the present apparatus. The amplified nucleic acid is a nucleic acid to be subjected to detection by the present apparatus.

### First Embodiment of Present Apparatus and Present Method

The following description is an example of a case in which the presence or absence of infection with any of four specific types of pathogens is determined from one specimen (saliva). Saliva may contain four types of target nucleic acids, each corresponding to a respective one of four specific pathogens. The four types of target nucleic acids are examples of a first nucleic acid, a second nucleic acid, a third nucleic acid, and a fourth nucleic acid in the present invention.

The present apparatus includes four types of reagents. Each reagent corresponds to each of the four types of target nucleic acids. Each reagent causes the corresponding target nucleic acid to be amplified. As a result, each reagent generates an amplified nucleic acid from the corresponding target nucleic acid. The four types of reagents are examples of a first reagent, a second reagent, a third reagent, and a fourth reagent in the present invention.

The reagent is disposed in a soft tube that serves as a supply line for saliva (hereinafter referred to as "tube"). The present apparatus includes four tubes. Each of the four types of reagents is disposed in a different tube. That is, each reagent disposed in each tube is different.

### Configuration of Present Apparatus (1)

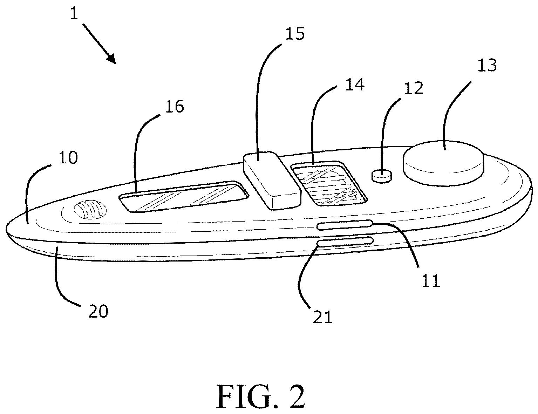

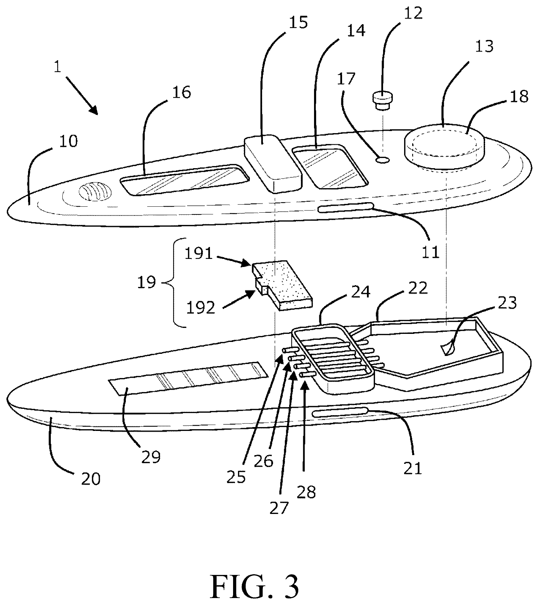

Fig. 2 is a perspective view of the present apparatus illustrating an embodiment of the present apparatus. Fig. 3 is an exploded perspective view of the present apparatus.

The present apparatus 1 includes a cover 10 and a base 20.

The cover 10 constitutes a housing of the present apparatus 1 together with the base 20. The cover 10 is disposed above the base 20 and is attached to the base 20. An internal space of the housing is sealed.

The cover 10 includes an upper heater insertion hole 11, a cap 12, a first button 13, a first window 14, a second button 15, a second window 16, a specimen introduction port 17, a developing solution tank 18, and an absorbent member 19.

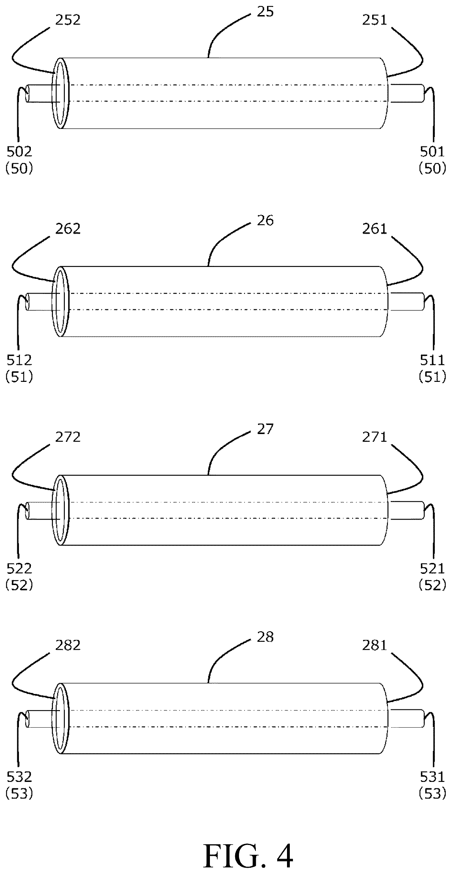

The base 20 includes a lower heater insertion hole 21, a specimen receiver 22, a needle 23, a tube mounting member 24, tubes 25 to 28, chromatography paper 29, and amplification members 50 to 53 (see Fig. 4).

The upper heater insertion hole 11 is a hole into which heating means for heating the tubes 25 to 28 is inserted. The upper heater insertion hole 11 is disposed on a side surface of the cover 10.

The cap 12 is a plug that seals the specimen introduction port 17. The cap 12 is an example of a sealing member according the present invention.

The first button 13 is a button for supplying the developing solution to the specimen receiver 22. The first button 13 includes the developing solution tank 18. When the first button 13 is pressed by a user, the first button 13 moves the developing solution tank 18 downward. The first button 13 is an example of a movement operating member in the present invention.

The first window 14 is a window that allows the inside of the cover 10 to be visible from outside the cover 10. The first window 14 is disposed above the tubes 25 to 28. A user can visually confirm, through the first window 14, whether the tubes 25 to 28 are being heated by the heating means. The first window 14 is an example of an amplification observation window in the present invention.

The second button 15 is a button for placing the absorbent member 19 between the tubes 25 to 28 and the chromatography paper 29. That is, the absorbent member 19 is placed between the tubes 25 to 28 and the chromatography paper 29. The second button 15 is an example of a supply operating member in the present invention.

The second window 16 is a window that allows the inside of the cover 10 to be visible from outside the cover 10. The second window 16 is disposed above the chromatography paper 29. That is, the second window 16 faces the chromatography paper 29. In other words, the second window 16 faces a color development surface described later. The second window 16 is disposed in the cover 10. A user can visually confirm whether the chromatography paper 29 is colored through the second window 16. The second window 16 is an example of a detection observation window in the present invention.

The specimen introduction port 17 is a port through which saliva to be placed in the specimen receiver 22 is introduced. The saliva is placed in the specimen receiver 22 from outside the present apparatus 1 through the specimen introduction port 17.

The developing solution tank 18 is a tank that stores the developing solution.

The absorbent member 19 supplies a detection solution generated in the tubes 25 to 28 to the chromatography paper 29. The absorbent member 19 is a material having high liquid absorbency (e.g., sponge) that absorbs the detection solution. The absorbent member 19 includes a first abutment portion 191 and a second abutment portion 192. The absorbent member 19 absorbs, from the first abutment portion 191, the detection solution generated in the tubes 25 to 28. The absorbent member 19 supplies the absorbed detection solution from the second abutment portion 192 to the chromatography paper 29. The absorbent member 19 is an example of a second supply line in the present invention.

The base 20 constitutes the housing of the present apparatus 1 together with the cover 10.

The lower heater insertion hole 21 is a hole into which the heating means for heating the tubes 25 to 28 is inserted. The lower heater insertion hole 21 is disposed on a side surface of the base 20. The lower heater insertion hole 21 is disposed below the upper heater insertion hole 11.

The specimen receiver 22 is a receptacle in which the saliva from the specimen introduction port 17 is placed and the developing solution from the developing solution tank 18 is supplied. The specimen receiver 22 is an example of a specimen placement member in the present invention.

The needle 23 is a needle that ruptures a portion of the developing solution tank 18. The needle 23 is an example of a rupturing member in the present invention.

The needle 23 constitutes, together with the first button 13, a discharge mechanism in the present invention. That is, in the present embodiment, the discharge mechanism includes the first button 13 and the needle 23.

The tube mounting member 24 is a member that mounts the tubes 25 to 28 to the base 20.

The tubes 25 to 28 are supply lines that supply the amplified nucleic acid toward the chromatography paper 29. The tubes 25 to 28 are reaction containers for the saliva and the reagent. The material of the tubes 25 to 28 is an elastomer such as rubber, polyurethane, or silicone, for example. The target nucleic acid contained in the saliva reacts with the reagent in each of the tubes 25 to 28 and is amplified. As a result, the amplified nucleic acid is generated in each of the tubes 25 to 28. The tubes 25 to 28 are examples of a first supply line in the present invention. The tubes 25 to 28 are examples of a first tubular body, a second tubular body, a third tubular body, and a fourth tubular body in the present invention.

The chromatography paper 29 is detection paper that detects the amplified nucleic acid. One end (unillustrated) of the chromatography paper 29 on a side of the second abutment portion 192 is an upstream end. The other end (unillustrated) of the chromatography paper 29 is a downstream end. The chromatography paper 29 includes a color developing agent, a capturing agent, and a color development surface. The chromatography paper 29 is an example of the test strip in the present invention.

The color developing agent is a colorant that causes the chromatography paper 29 to develop color. The color developing agent binds to the amplified nucleic acid. The chromatography paper 29 includes, for each amplified nucleic acid to be bound, a color developing agent configured to bind to the amplified nucleic acid. The chromatography paper 29 includes four color developing agents. Each of the four color developing agents is disposed at a different position on the chromatography paper 29. The color developing agent is disposed on an upstream end side relative to the capturing agent. The color developing agent in the present embodiment is colored beads. The four color developing agents are examples of a first color developing agent, a second color developing agent, a third color developing agent, and a fourth color developing agent in the present invention.

The capturing agent is a substance that captures the amplified nucleic acid. The capturing agent captures the amplified nucleic acid bound to the color developing agent. The chromatography paper 29 includes, for each amplified nucleic acid to be captured, a capturing agent configured to capture the amplified nucleic acid. The chromatography paper 29 includes four capturing agents. Each of the four capturing agents is disposed at a different position on the chromatography paper 29. The capturing agent is disposed on a downstream end side relative to the color developing agent. The four capturing agents are examples of a first capturing agent, a second capturing agent, a third capturing agent, and a fourth capturing agent in the present invention.

The color development surface is a surface of the chromatography paper 29 on which the capturing agent is disposed. The chromatography paper 29 includes four color development surfaces. Each of the four color development surfaces corresponds to each of the four capturing agents. The color development surface exhibits color (i.e., develops color) when the amplified nucleic acid is captured by the capturing agent. That is, when the color development surface exhibits color, the chromatography paper 29 exhibits color.

Fig. 4 is a schematic diagram illustrating a state in which the amplification members 50 to 53 are extended through the tubes 25 to 28, respectively. The figure illustrates that the amplification members 50 to 53 are disposed in the through holes of the tubes 25 to 28, respectively.

Each of the tubes 25 to 28 has substantially the same shape and the same function. In the following description, the shape and function of the tubes 25 to 28 will be described by using the tube 25 as an example.

The tube 25 is a tubular body. The tube 25 has a through hole. The tube 25 includes a first opening 251 and a second opening 252. The first opening 251 is an opening disposed on a side of the specimen receiver 22. That is, the tube 25 is coupled to the specimen receiver 22. The second opening 252 is an opening disposed on the chromatography paper 29 side.

Each of the amplification members 50 to 53 has substantially the same shape and the same function, except that the immobilized reagents are different. In the following description, the shape and function of the amplification members 50 to 53 will be described by using the amplification member 50 as an example.

The amplification member 50 is a member on which the reagent is immobilized. The reagent immobilized on the amplification member 50 is a reagent for generating the amplified nucleic acid from the target nucleic acid. The reagent immobilized on the amplification member 50 is in a dry state. The reagent in a dry state can be stored at ambient temperature. Thus, the present apparatus 1 can be stored at ambient temperature. The amplification member 50 includes a support that carries the reagent. The support is an aggregate of absorbent materials such as glass fibers.

The amplification member 50 is a linear body. The amplification member 50 is extended through the through hole of the tube 25. That is, the amplification member 50 is disposed in the tube 25. The amplification member 50 includes one end 501 and the other end 502. The one end 501 is an end portion of the amplification member 50. The one end 501 is disposed on a side of the first opening 251. The one end 501 is exposed outside the tube 25 from the first opening 251. The other end 502 is an end portion of the amplification member 50. The other end 502 is disposed on a side of the second opening 252. The other end 502 is exposed outside the tube 25 from the second opening 252.

As described above, the amplification members 50 to 53 that are linear bodies are disposed in the tubes 25 to 28, respectively. The amplification members 50 to 53 disposed in the tubes 25 to 28 are examples of a first linear body, a second linear body, a third linear body, and a fourth linear body in the present invention.

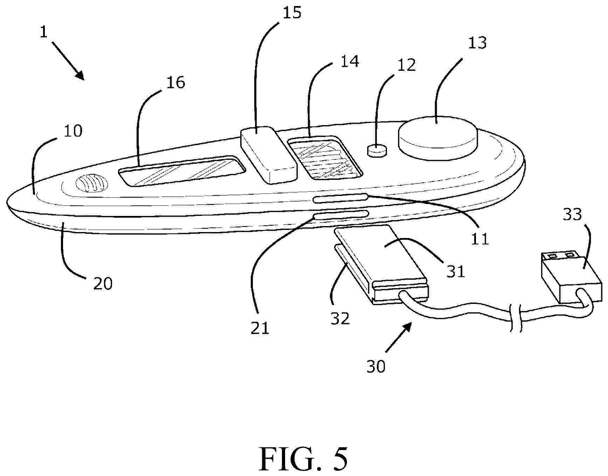

Fig. 5 is a schematic diagram illustrating a state before the heater 30 is attached to the present apparatus 1. The figure illustrates that the heater 30 is attached to the present apparatus 1.

The heater 30 is an apparatus that heats a target substance. The heater 30 is an example of the heating means in the present invention.

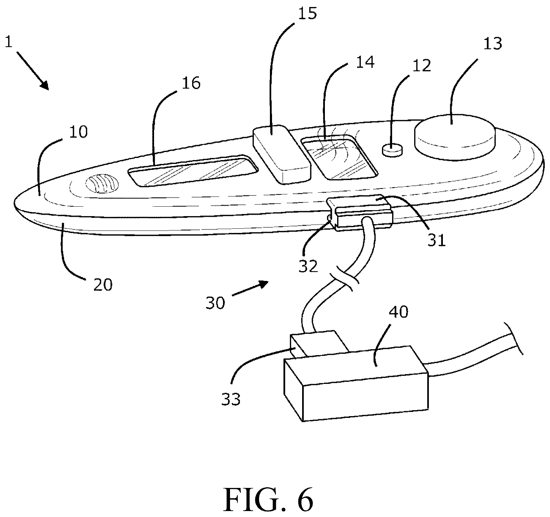

The heater 30 includes an upper heater 31, a lower heater 32, and a power connector 33. The upper heater 31 is inserted into the upper heater insertion hole 11. The lower heater 32 is inserted into the lower heater insertion hole 21. The power connector 33 is a terminal connected to a power supply 40 (see Fig. 6). The power connector 33 is a USB connector.

Fig. 6 is a schematic diagram illustrating a state after the heater 30 is attached to the present apparatus 1. The figure illustrates that the heater 30 is attached to the present apparatus 1. The figure illustrates that the power connector 33 is connected to the power supply 40.

The power supply 40 is a power source that supplies power to the heater 30. The power supply 40 is a USB power supply.

### Operation of Present Apparatus (1)

The following description is an example of a case in which a subject is infected with one type of pathogen among the four specific types of pathogens, i.e., a case in which the saliva collected from the subject contains a nucleic acid derived from one type of specific pathogen.

First, the cap 12 is removed from the specimen introduction port 17.

Then, the saliva is placed in the specimen receiver 22 through the specimen introduction port 17. The saliva is collected by a user in advance and accommodated in a predetermined container. The user places the saliva accommodated in the container in the specimen receiver 22 by a predetermined method (e.g., pipetting).

Then, the cap 12 is attached to the specimen introduction port 17.

Then, the heater 30 is attached to the present apparatus 1. That is, the upper heater 31 is inserted into the upper heater insertion hole 11. The lower heater 32 is inserted into the lower heater insertion hole 21. The tube mounting member 24 is disposed between the upper heater 31 and the lower heater 32.

Then, the saliva placed in the specimen receiver 22 penetrates into the one ends 501 to 531 of the amplification members 50 to 53 and portions in the vicinities thereof, which are exposed outside the tubes 25 to 28, respectively. The saliva penetrated into the one ends 501 to 531 of the amplification members 50 to 53 and the portions in the vicinities thereof is absorbed into the tubes 25 to 28, respectively. The tubes 25 to 28 mounted to the tube mounting member 24 are heated by the heater 30. That is, the saliva absorbed into the tubes 25 to 28 is heated by the heater 30.

Then, the saliva absorbed into the tubes 25 to 28 moves from the side of the one ends 501 to 531 toward the side of the other ends 502 to 532. The movement of the saliva through the tubes 25 to 28 is caused by the capillary action of the tubes 25 to 28. The saliva moving through the tubes 25 to 28 is mixed with the reagents immobilized on the amplification members 50 to 53.

The saliva moving through the tubes 25 to 28 is heated by the heater 30. The heater 30 performs a heating operation to reach a temperature suitable for amplification of the target nucleic acid contained in the saliva in the tubes 25 to 28 (e.g., 65°C). Herein, the heating operation of the heater 30 starts before the saliva is placed in the specimen receiver 22, for example. The target nucleic acid contained in the heated saliva is amplified. As a result, the amplified nucleic acid is generated.

In this way, the saliva moving through the tubes 25 to 28 slowly diffuses into the amplification members 50 to 53, which are supports that carry the reagents. The saliva diffused in the amplification members 50 to 53 is mixed with the reagents immobilized on the amplification members 50 to 53. As a result, the target nucleic acid contained in the saliva is amplified. That is, the amplification of the target nucleic acid in the present apparatus 1 does not require stirring the reagent or performing centrifugation.

Then, the first button 13 is pressed by the user. The developing solution tank 18 accommodated in the first button 13 moves downward toward the needle 23 together with the first button 13. The developing solution tank 18 is brought into contact with the needle 23. The developing solution tank 18 is ruptured when brought into contact with the needle 23. When the developing solution tank 18 is ruptured, the developing solution stored in the developing solution tank 18 is discharged from the developing solution tank 18. The discharged developing solution is supplied to the specimen receiver 22.

Herein, the timing at which the first button 13 is pressed by the user is related to the amplification time of the target nucleic acids disposed in the tubes 25 to 28. The amplification time of the target nucleic acids is the time required for the target nucleic acids to be amplified to generate detectable amplified nucleic acids. That is, when the amplification time of the target nucleic acids is assumed to be 15 minutes, the user presses the first button 13 after 15 minutes have elapsed since the user placed the saliva in the specimen receiver 22.

Then, the developing solution supplied to the specimen receiver 22 penetrates into the one ends 501 to 531 of the amplification members 50 to 53 and the portions in the vicinities thereof, which are exposed outside the tubes 25 to 28 from the first openings 251 to 281, respectively. The developing solution penetrated into the one ends 501 to 531 of the amplification members 50 to 53 and the portions in the vicinities thereof is absorbed into the tubes 25 to 28, respectively.

Then, the developing solution absorbed into the tubes 25 to 28 moves from the side of the one ends 501 to 531 toward the side of the other ends 502 to 532. The movement of the developing solution through the tubes 25 to 28 is caused by the capillary action of the tubes 25 to 28. The developing solution moving through the tubes 25 to 28 is mixed with the amplification solution in the tubes 25 to 28. As a result, the detection solution is generated.

Then, the second button 15 is pressed by the user. The absorbent member 19 held by the second button 15 moves downward together with the second button 15. As a result, the absorbent member 19 is placed between the tubes 25 to 28 and the chromatography paper 29.

The absorbent member 19 placed between the tubes 25 to 28 and the chromatography paper 29 remains placed therebetween even after the pressing of the second button 15 has been released.

Herein, the timing at which the second button 15 is pressed by the user (hereinafter referred to as "second timing") is related to the timing at which the first button 13 is pressed by the user (hereinafter referred to as "first timing"). The second timing is from the first timing until a predetermined time (e.g., 3 minutes) has elapsed.

The first abutment portion 191 of the absorbent member 19 placed between the tubes 25 to 28 and the chromatography paper 29 abuts the amplification members 50 to 53. The first abutment portion 191 abuts the whole or part of the exposed portions of the amplification members 50 to 53 from the second openings 252 to 282, respectively.

The second abutment portion 192 of the absorbent member 19 placed between the tubes 25 to 28 and the chromatography paper 29 abuts the chromatography paper 29. The second abutment portion 192 abuts one end of the chromatography paper 29 on the second abutment portion 192 side and a portion vicinity thereof.

Then, the absorbent member 19 absorbs the detection solution in the amplification members 50 to 53 from the first abutment portion 191 abutting the amplification members 50 to 53.

Then, the detection solution absorbed into the absorbent member 19 is supplied to the chromatography paper 29 from the second abutment portion 192 abutting the chromatography paper 29.

Then, the detection solution supplied to the chromatography paper 29 moves on the chromatography paper 29 from the upstream end toward the downstream end. The detection solution is mixed with the color developing agent disposed on the upstream end side of the chromatography paper 29.

Then, the amplified nucleic acid in the detection solution mixed with the color developing agent binds to the color developing agent.

Then, the amplified nucleic acid bound to the color developing agent moves toward the downstream end on the chromatography paper 29. The amplified nucleic acid moving through the chromatography paper 29 is mixed with the capturing agent disposed on the color development surface of the chromatography paper 29.

Then, the amplified nucleic acid mixed with the capturing agent binds to the capturing agent. The amplified nucleic acid includes a site that binds to the color developing agent and a site that binds to the capturing agent. The site that binds to the color developing agent is different from the site that binds to the capturing agent.

Then, the amplified nucleic acid bound to the capturing agent is immobilized on the color development surface (the chromatography paper 29) via the capturing agent.

Then, the color development surface develops color through the color developing agent that binds to the amplified nucleic acid immobilized on the color development surface.

The colored color development surface constitutes the chromatography paper 29. The chromatography paper 29 having the colored color development surface is visible through the second window 16.

The user confirms that one of the four color development surfaces of the chromatography paper 29 has developed color. That is, the user determines that the subject is infected with one type of pathogen corresponding to the color development surface that has developed color among the four types of pathogens to be detected by the present apparatus 1.

### Conclusion (1)

As described above, the present apparatus 1 includes the specimen receiver 22, the tubes 25 to 28, the chromatography paper 29, and the amplification members 50 to 53. The amplification members 50 to 53 are disposed in the tubes 25 to 28, respectively. The saliva placed in the specimen receiver 22 is absorbed into the tubes 25 to 28. The saliva absorbed into the tubes 25 to 28 is mixed with the reagents immobilized in the amplification members 50 to 53 disposed in the tubes 25 to 28, respectively. That is, the saliva is placed in the specimen receiver 22. As a result, the saliva is mixed with the reagents. Herein, the saliva placed in the specimen receiver 22 is absorbed into the tubes 25 to 28 due to the capillary action of the tubes 25 to 28. In other words, the saliva placed in the specimen receiver 22 is mixed with the specimen without any operation by a user. That is, the present apparatus 1 is capable of mixing saliva with the specimen without relying on the user's expertise or skill.

As described above, the present apparatus 1 includes the second button 15 and the absorbent member 19. When the second button 15 is pressed, the absorbent member 19 is placed between the tubes 25 to 28 and the chromatography paper 29. The absorbent member 19 placed between the tubes 25 to 28 and the chromatography paper 29 absorbs the detection solution from the tubes 25 to 28. The absorbent member 19 that has absorbed the detection solution supplies the detection solution to the chromatography paper 29. That is, when the second button 15 is pressed, the detection solution is supplied from the tubes 25 to 28 to the chromatography paper 29. In other words, the detection solution is supplied to the chromatography paper 29 solely by the pressing of the second button 15 by the user. That is, the present apparatus 1 is capable of supplying the detection solution to the chromatography paper 29 without relying on the user's expertise or skill.

As described above, the present apparatus 1 includes the housing (i.e., the cover 10 and the base 20), the cap 12, and the specimen introduction port 17. The housing accommodates the absorbent member 19, the specimen receiver 22, the tubes 25 to 28, the chromatography paper 29, and the amplification members 50 to 53. The cap 12 seals the specimen introduction port 17. The specimen introduction port 17 is a port through which the specimen to be placed in the specimen receiver 22 is introduced. The specimen introduction port 17 is disposed in the cover 10. The cap 12 is removed from the specimen introduction port 17 when the saliva is being placed in the specimen receiver 22. The cap 12 is attached to the specimen introduction port 17 after the saliva has been placed in the specimen receiver 22 through the specimen introduction port 17. When the specimen introduction port 17 is not sealed by (i.e., not attached to) the cap 12, the inside of the housing communicates with the outside of the housing solely through the specimen introduction port 17. That is, the inside of the housing communicates with the outside only when the saliva is introduced through the specimen introduction port 17. In other words, the inside of the present apparatus 1 is in a sealed state except when the saliva is being introduced (i.e., when the cap 12 is removed from the specimen introduction port 17).

As described above, the present apparatus 1 includes the second window 16. The second window 16 allows the chromatography paper 29 to be visible to a user from outside the housing. That is, the chromatography paper 29 is observed by the user through the second window 16. In other words, the user can observe the presence or absence of coloration of the chromatography paper 29 through the second window 16. The user can determine whether nucleic acids derived from a specific pathogen are contained in the saliva collected from the subject, based on the presence or absence of coloration of the chromatography paper 29.

As described above, the present apparatus 1 includes the first button 13, the developing solution tank 18, and the needle 23. The first button 13 constitutes the discharge mechanism together with the needle 23. The first button 13 moves the developing solution tank 18 toward the needle 23. The developing solution tank 18 stores the developing solution that supplies the amplified nucleic acid toward the chromatography paper 29. The needle 23 ruptures a portion of the developing solution tank 18 when the first button 13 is pressed and the developing solution tank 18 is moved. When the needle 23 ruptures a portion of the developing solution tank 18, the developing solution is discharged from the developing solution tank 18. That is, the developing solution is discharged from the developing solution tank 18 when the first button 13 is pressed by the user. The developing solution discharged from the developing solution tank 18 is supplied to the tubes 25 to 28. The developing solution supplied to the tubes 25 to 28 is mixed with the amplification solution in the tubes 25 to 28. That is, the developing solution is mixed with the amplification solution solely by the pressing of the first button 13 by the user. In other words, the present apparatus 1 is capable of mixing the developing solution and the amplification solution without relying on the user's expertise or skill.

As described above, the reagents include the first reagent that amplifies the corresponding nucleic acid and the second reagent that amplifies the corresponding nucleic acid. The linear bodies include the first linear body disposed inside the corresponding tube body and the second linear body disposed inside the corresponding tube body. The first reagent is immobilized on the first linear body. The second reagent is immobilized on the second linear body. That is, the first linear body and the second linear body are disposed inside the corresponding tube bodies, respectively. As a result, the present apparatus 1 is capable of simultaneously amplifying the target nucleic acids contained in the saliva by means of the plurality of reagents.

As described above, the target nucleic acids include the first nucleic acid and the second nucleic acid. The reagents include the first reagent that amplifies the corresponding nucleic acid and the second reagent that amplifies the corresponding nucleic acid. The first reagent amplifies the first nucleic acid. The second reagent amplifies the second nucleic acid. That is, the present apparatus 1 is capable of simultaneously amplifying the nucleic acids derived from a plurality of specific pathogens. As a result, the present apparatus 1 is capable of simultaneously detecting the presence or absence of infection with any of the plurality of specific pathogens.

As described above, the present apparatus 1 includes the second button 15 and the absorbent member 19. The absorbent member 19 includes the first abutment portion 191 and the second abutment portion 192. The first abutment portion 191 abuts the amplification members 50 to 53. The second abutment portion 192 abuts the chromatography paper 29. When the second button 15 is pressed, the absorbent member 19 is placed between the chromatography paper 29 and the amplification members 50 to 53. The detection solution generated by the amplification members 50 to 53 is supplied from the first abutment portion 191 to the second abutment portion 192. The detection solution is supplied from the second abutment portion 192 to the chromatography paper 29. That is, when the second button 15 is pressed, the detection solution absorbed into the absorbent member 19 is supplied to the chromatography paper 29. In other words, the present apparatus 1 is capable of supplying the detection solution to the chromatography paper 29 without relying on the user's expertise or skill.

Note that, in the above description, the number of target nucleic acids to be detected by the present apparatus 1 is four. However, the number of target nucleic acids to be detected by the present apparatus is not limited thereto. That is, for example, the number of target nucleic acids detected by the present apparatus may be one or a plurality (excluding four).

In the above description, the number of reagents included in the present apparatus 1 coincides with the number of target nucleic acids to be detected by the present apparatus 1. However, the number of reagents included in the present apparatus does not necessarily coincide with the number of target nucleic acids to be detected by the present apparatus. That is, for example, the present apparatus may include a plurality of reagents corresponding to one type of target nucleic acid to be detected by the present apparatus. That is, the number of reagents included in the present apparatus is appropriately set according to the number of target nucleic acids to be detected by the present apparatus.

In the above description, the number of linear bodies included in the present apparatus 1 coincides with the number of reagents included in the present apparatus 1. However, the number of linear bodies included in the present apparatus does not necessarily coincide with the number of reagents included in the present apparatus. That is, for example, the present apparatus may include one linear body on which a plurality of reagents is immobilized.

### Second Embodiment of Present Apparatus and Present Method

Another embodiment (a second embodiment) of the present apparatus and the present method are described below with a focus on differences from the embodiment (the first embodiment) of the present method described above.

The second embodiment differs from the first embodiment in that the number of first supply lines is one, whereas a plurality of first supply lines is provided in the first embodiment. The second embodiment differs from the first embodiment in that a portion of the first supply line is exposed outside the housing, whereas the entire first supply lines are accommodated in the housing in the first embodiment. The second embodiment differs from the first embodiment in that the developing solution tank is disposed in the base, whereas the developing solution tank is disposed in the cover in the first embodiment.

The following description is an example of a case in which the presence or absence of infection with one specific type of pathogen is determined from one specimen (saliva). Saliva may contain one type of target nucleic acid corresponding to one specific type of pathogen. The present apparatus includes one type of reagent. The reagent corresponds to the one type of target nucleic acid. The reagent generates an amplified nucleic acid from the corresponding target nucleic acid. The reagent is disposed in a tube that serves as a supply line for saliva. The present apparatus includes one tube. The one type of reagent is disposed in the one tube.

### Configuration of Present Apparatus (2)

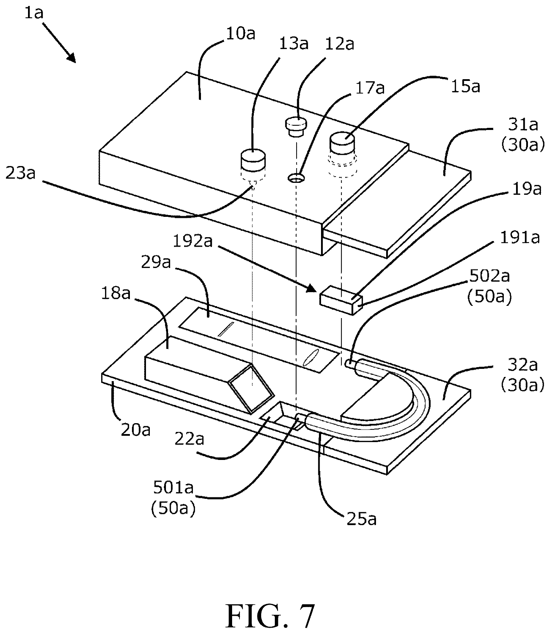

Fig. 7 is an exploded perspective view of the present apparatus illustrating another embodiment of the present apparatus.

The present apparatus 1a includes a cover 10a and a base 20a.

The cover 10a includes a cap 12a, a first button 13a, a second button 15a, a second window (not illustrated), a specimen introduction port 17a, an absorbent member 19a, and a needle 23a.

The base 20a includes a developing solution tank 18a, a specimen receiver 22a, a tube 25a, a chromatography paper 29a, and an amplification member 50a.

The cover 10a, the cap 12a, the first button 13a, the second button 15a, the second window (not illustrated), the specimen introduction port 17a, the developing solution tank 18a, the absorbent member 19a, the base 20a, the specimen receiver 22a, the needle 23a, the tube 25a, the chromatography paper 29a, and the amplification member 50a correspond to the cover 10, the cap 12, the first button 13, the second button 15, the second window 16, the specimen introduction port 17, the developing solution tank 18, the absorbent member 19, the base 20, the specimen receiver 22, the needle 23, the tube 25, the chromatography paper 29, and the amplification member 50 in the first embodiment, respectively.

Unlike the cover 10, the cover 10a does not include the developing solution tank 18a. Unlike the cover 10, the cover 10a includes the needle 23a.

Unlike the first button 13, the first button 13a does not include the developing solution tank 18a. Unlike the first button 13, the first button 13a includes the needle 23a. When the first button 13a is pressed by a user, the first button 13a moves the needle 23a downward. The first button 13a is an example of a movement operating member in the present invention.

Unlike the base 20, the base 20a includes the developing solution tank 18a. Unlike the base 20, the base 20a does not include the needle 23a.

Unlike the tubes 25 to 28, a portion of the tube 25a is exposed outside the housing (i.e., the cover 10a and the base 20a). Both ends of the tube 25a and portions in the vicinity thereof are accommodated in the housing. The remaining portion of the tube 25a is exposed outside the housing so as to be heated by the heating means.

Unlike the chromatography paper 29, the chromatography paper 29a has one color development surface.

### Operation of Present Apparatus (2)

The following description is an example of a case in which a subject is infected with one specific type of pathogen, that is, a case in which saliva collected from the subject contains a nucleic acid derived from one specific type of pathogen.

First, the cap 12a is removed from the specimen introduction port 17a.

Then, the saliva is placed in the specimen receiver 22a through the specimen introduction port 17a.

Then, the cap 12a is attached to the specimen introduction port 17a.

Then, the heater 30a is attached to the present apparatus 1a. That is, the upper heater 31a is disposed above the tube 25a. The lower heater 32a is disposed below the tube 25a. The tube 25a is heated by the heater 30a.

Then, the saliva placed in the specimen receiver 22a penetrates into one end 501a of the amplification member 50a and a portion in the vicinity thereof, which is exposed outside the tube 25a. The saliva penetrated into the one end 501a of the amplification member 50a and the portion in the vicinity thereof is absorbed into the tube 25a.

Then, the saliva absorbed into the tube 25a moves from the side of the one end 501a toward the side of the other end 502a. The movement of the saliva through the tube 25a is caused by the capillary action of the tube 25a. The saliva moving through the tube 25a is mixed with the reagent immobilized on the amplification member 50a.

The saliva moving through the tube 25a is heated by each of the upper heater 31a and the lower heater 32a (hereinafter collectively referred to as "upper and lower heaters"). The upper and lower heaters are apparatuses that heat a target substance. The upper and lower heaters are examples of the heating means in the present invention. The upper and lower heaters perform a heating operation to reach a temperature suitable for amplification of a target nucleic acid contained in the saliva in the tube 25a (e.g., 65 ° C). The target nucleic acid contained in the heated saliva is amplified. As a result, an amplified nucleic acid is generated.

Then, the first button 13a is pressed by the user. The needle 23a mounted to the first button 13a moves downward toward the developing solution tank 18a together with the first button 13a. The needle 23a is brought into contact with the developing solution tank 18a. The developing solution tank 18a is ruptured when the tank 18a is brought into contact with the needle 23a. When the developing solution tank 18a is ruptured, the developing solution stored in the developing solution tank 18a is discharged from the developing solution tank 18a. The discharged developing solution is supplied to the specimen receiver 22a

Then, the developing solution supplied to the specimen receiving 22a penetrates the one end 501a of the amplification member 50a and the portion in the vicinity thereof, which is exposed outside the tube 25a. The developing solution penetrated into the one end 501a of the amplification member 50a and the portion in the vicinity thereof is absorbed into the tube 25a.

Then, the developing solution absorbed into the tube 25a moves from the side of the one end 501a toward the side of the other end 502a. The movement of the developing solution through the tube 25a is caused by the capillary action of the tube 25a. The developing solution moving through the tube 25a is mixed with the amplification solution in the tube 25a. As a result, the detection solution is generated.

Then, the second button 15a is pressed by the user. The absorbent member 19a held by the second button 15a moves downward together with the second button 15a and is placed between the tube 25a and the chromatography paper 29a.

The absorbent member 19a placed between the tube 25a and the chromatography paper 29a remains placed therebetween even after the pressing of the second button 15a has been released.

A first abutment portion 191a of the absorbent member 19a placed between the tube 25a and the chromatography paper 29a abuts the amplification member 50a. The first abutment portion 191a abuts the whole or part of the exposed portions of the amplification member 50a from the second opening (not illustrated).

A second abutment portion 192a of the absorbent member 19a placed between the tube 25a and the chromatography paper 29a abuts the chromatography paper 29a. The second abutment portion 192a abuts one end of the chromatography paper 29a on the side of the second abutment portion 192a and a portion in the vicinity of the one end.

Then, the absorbent member 19a absorbs the detection solution in the amplification member 50a from the first abutment portion 191a abutting the amplification member 50a.

Then, the detection solution absorbed into the absorbent member 19a is supplied to the chromatography paper 29a from the second abutment portion 192a abutting the chromatography paper 29a.

Then, the detection solution supplied to the chromatography paper 29a moves on the chromatography paper 29a from the upstream end toward the downstream end. The detection solution is mixed with the color developing agent disposed on the upstream end side of the chromatography paper 29a.

Then, the amplified nucleic acid in the detection solution mixed with the color developing agent binds to the color developing agent.

Then, the amplified nucleic acid bound to the color developing agent moves on the chromatography paper 29a toward the downstream end. The amplified nucleic acid moving through the chromatography paper 29a is mixed with the capturing agent disposed on the color development surface of the chromatography paper 29a.

Then, the amplified nucleic acid mixed with the capturing agent binds to the capturing agent.

Then, the amplified nucleic acid bound to the capturing agent is immobilized on the color development surface (the chromatography paper 29a) via the capturing agent.

Then, the color development surface develops color through the color developing agent that binds to the amplified nucleic acid immobilized on the color development surface.

The colored surface constitutes the chromatography paper 29a. The chromatography paper 29a having the colored surface is visible through the second window (unillustrated) disposed in the cover 10a.

The user confirms that the color development surface of the chromatography paper 29a has developed color. That is, the user determines that the subject is infected with one type of pathogen to be detected by the present apparatus 1a.

### Conclusion (2)

As described above, the present apparatus 1a includes the housing (i.e., the cover 10a and the base 20a) and the tube 25a. A portion of the tube 25a is accommodated in the housing. The remaining portion of the tube 25a is exposed outside the housing so as to be heated by the heating means. Since the remaining portion of the tube 25a is exposed outside the housing, the variety of heating means available for heating the tube 25a increases.

As described above, since the remaining portion of the tube 25a is exposed outside the housing, the downsizing of the present apparatus 1a is easier than the downsizing of the present apparatus 1 in which the tube 25a is accommodated in the housing.

Note that, in the above description, the number of target nucleic acids to be detected by the present apparatus 1a is one. However, the number of target nucleic acids to be detected by the present apparatus is not limited to one. That is, for example, the number of target nucleic acids to be detected by the present apparatus may be two or more.

In the above description, the number of reagents included in the present apparatus 1a coincides with the number of target nucleic acids to be detected by the present apparatus 1a. However, the number of reagents included in the present apparatus is not limited thereto. That is, for example, the present apparatus may include a plurality of reagents corresponding to one type of target nucleic acid to be detected by the present apparatus. Accordingly, the number of reagents included in the present apparatus is appropriately set according to the number of target nucleic acids to be detected by the present apparatus.

In the above description, the number of the linear bodies included in the present apparatus 1a coincides with the number of the reagents included in the present apparatus 1a. However, the number of linear bodies included in the present apparatus is not limited thereto. That is, for example, the present apparatus may include one linear body on which a plurality of reagents is immobilized.

### Other Embodiments

Other embodiments (a third embodiment to a fifth embodiment) of the present apparatus and the present method are described below with a focus on differences from the embodiments (the first embodiment and the second embodiment) of the present apparatus and the present method described above.

In the following description, when the present apparatus 1 and the present apparatus 1a are not particularly distinguished from each other, each of the present apparatus 1 and the present apparatus 1a is referred to as a present apparatus 1x.

In the following description, when the second button 15 and the second button 15a are not particularly distinguished from each other, each of the second button 15 and the second button 15a is referred to as a second button 15x.

In the following description, when the absorbent member 19 and the absorbent member 19a are not particularly distinguished from each other, each of the absorbent member 19 and the absorbent member 19a is referred to as an absorbent member 19x.

In the following description, when the base 20 and the base 20a are not particularly distinguished from each other, each of the base 20 and the base 20a is referred to as a base 20x.

In the following description, when the specimen receiver 22 and the specimen receiver 22a are not particularly distinguished from each other, each of the specimen receiver 22 and the specimen receiver 22a is referred to as a specimen receiver 22x.

In the following description, when the tubes 25 to 28 and the tube 25a are not particularly distinguished from each other, each of the tubes 25 to 28 and the tube 25a is referred to as a tube 25x.

In the following description, when the chromatography paper 29 and the chromatography paper 29a are not particularly distinguished from each other, each of the chromatography paper 29 and the chromatography paper 29a is referred to as a chromatography paper 29x.

In the following description, when the amplification members 50 to 53 and the amplification member 50a are not particularly distinguished from each other, each of the amplification members 50 to 53 and the amplification member 50a is referred to as an amplification member 50x.

In the following description, the "downstream direction" is a direction in which the amplified nucleic acid is supplied to the chromatography paper. The "upstream direction" is a direction opposite to the downstream direction. The "supply direction" collectively refers to the upstream direction and the downstream direction.

In the following description, the tube 25x is connected to the specimen receiver 22x.

In the following description, the amplification member 50x is disposed in the tube 25x.

### Differences between First and Second Embodiments and Third to Fifth Embodiments

### First and Second Embodiments

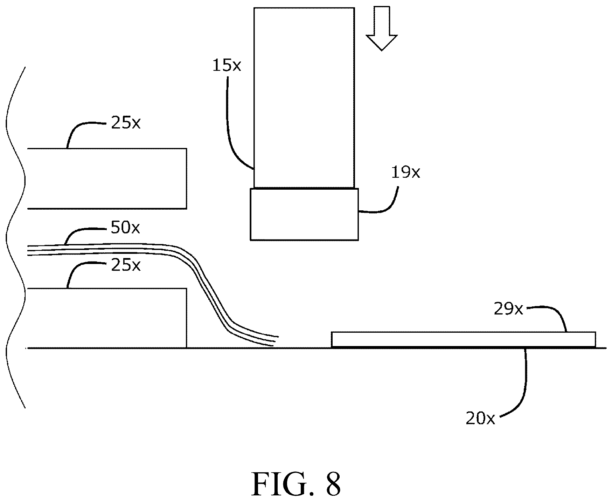

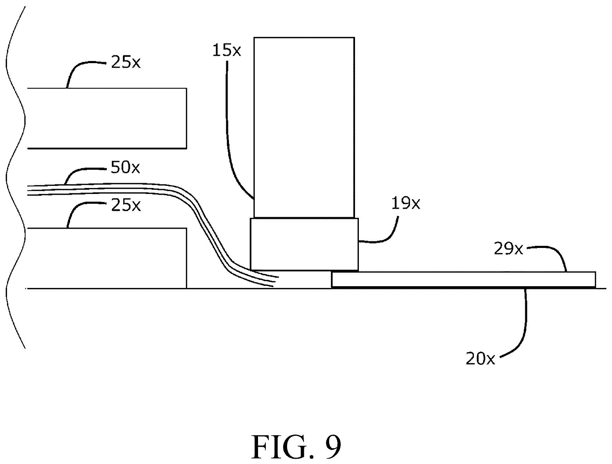

Fig. 8 is a schematic diagram illustrating a state in which the second button 15x is being operated. The figure illustrates that the absorbent member 19x is held by the second button 15x. In the figure, a white arrow indicates a moving direction of the second button 15x when the second button 15x is operated (i.e., when the second button 15x is pressed by a user). Fig. 9 is a schematic diagram illustrating a state in which the amplification member 50x is connected to the chromatography paper 29x.

In the first and second embodiments, the present apparatus 1x includes the second button 15x, the absorbent member 19x, the base 20x, the tube 25x, the chromatography paper 29x, and the amplification member 50x. The second button 15x is disposed above the amplification member 50x.

The absorbent member 19x is placed between the tube 25x and the chromatography paper 29x when the second button 15x is operated.

The absorbent member 19x placed between the tube 25x and the chromatography paper 29x abuts the amplification member 50x and the chromatography paper 29x. That is, the absorbent member 19x connects the amplification member 50x to the chromatography paper 29x. In other words, when the second button 15x is operated, the amplification member 50x is connected to the chromatography paper 29x via the absorbent member 19x. The absorbent member 19x abutting the amplification member 50x absorbs, from the amplification member 50x, the detection solution in the amplification member 50x. The detection solution absorbed from the amplification member 50x into the absorbent member 19x is supplied to the chromatography paper 29x from the absorbent member 19x.

In this way, the amplified nucleic acid is supplied to the chromatography paper 29x when the second button 15x is operated. In other words, the second button 15x causes the amplified nucleic acid to be supplied to the chromatography paper 29x.

### Third to Fifth Embodiments

The third to fifth embodiments differ from the first and second embodiments in that the second supply line is attached to one end portion of the test strip on the upstream side, whereas the second supply line is held by the supply operating member in the first and second embodiments.

The third and fourth embodiments differ from the first and second embodiments in that the present apparatus includes a placement table, whereas the present apparatus does not include the placement table in the first and second embodiments. Details of the placement table will be described later.

The third embodiment differs from the first and second embodiments in that the supply operating member presses the second supply line, whereas the second supply line is disposed between the first supply line and the test strip by the supply operating member in the first and second embodiments.

The fourth embodiment differs from the first and second embodiments in that the present apparatus includes a connecting member, whereas the present apparatus does not include the connecting member in the first and second embodiments. Details of the connecting member will be described later.

The fourth embodiment differs from the first and second embodiments in that the supply operating member causes the amplification member to be connected to the second supply line, whereas the second supply line is disposed between the first supply line and the test strip by the supply operating member in the first and second embodiments.

The fifth embodiment differs from the first and second embodiments in that the supply operating member is attached to the other end portion of the test strip on the downstream side, whereas the supply operating member is mounted (disposed) to the housing in the first and second embodiments.

The fifth embodiment differs from the first and second embodiments in that the supply operating member presses the second supply line, whereas the second supply line is disposed between the first supply line and the test strip by the supply operating member in the first and second embodiments.

In the following description of the third to fifth embodiments, the same names as those in the first and second embodiments are used for the members corresponding to those in the first and second embodiments for convenience of description.

In the following description of the third to fifth embodiments, the same members and the members with a common function as in the first and second embodiments are indicated with the same reference signs as in the first and second embodiments for convenience of description.

In the following description of the third to fifth embodiments, the detection solution is generated in the amplification member 50x disposed in the tube 25x. The detection solution is disposed in the amplification member 50x in the tube 25x.

### Third Embodiment of Present Apparatus and Present Method

The third embodiment of the present apparatus and the present method will be described below.

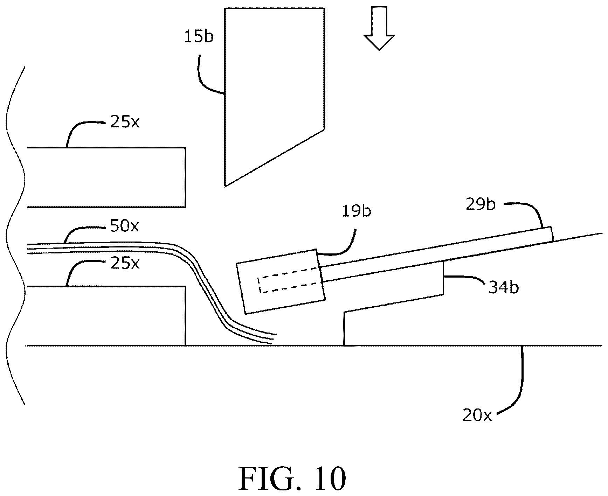

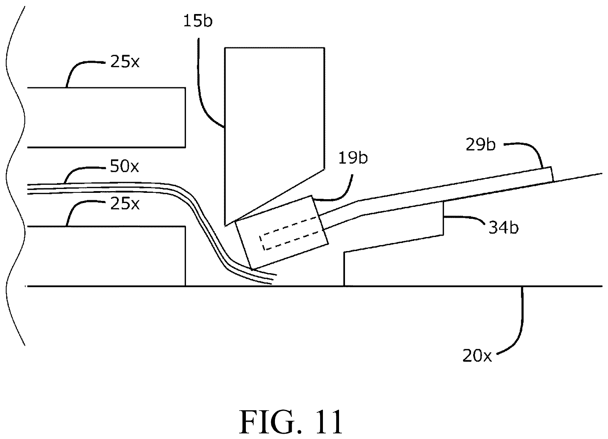

Fig. 10 is a schematic diagram illustrating yet another embodiment of the present apparatus and illustrating a state in which a second button 15b is being operated. In the figure, a white arrow indicates a moving direction of the second button 15b when the second button 15b is operated (i.e., when the second button 15b is pressed by a user). Fig. 11 is a schematic diagram illustrating a state in which the amplification member 50x is connected to a chromatography paper 29b.

The second button 15b is a button for pressing an absorbent member 19b. The second button 15b is disposed above the amplification member 50x. The second button 15b is disposed above the absorbent member 19b. The second button 15b is an example of the supply operating member in the present invention.

The absorbent member 19b supplies the detection solution generated in the amplification member 50x in the tube 25x to the chromatography paper 29b. The absorbent member 19b is attached to one end portion (not illustrated) of the chromatography paper 29b on the upstream side in the supply direction of the amplified nucleic acid. The absorbent member 19b is disposed between the amplification member 50x and the second button 15b in the vertical direction. Details of the one end portion will be described later.

Herein, the second button 15b presses the absorbent member 19b. That is, when the second button 15b is operated, the absorbent member 19b is pressed toward the amplification member 50x by the second button 15b. The absorbent member 19b is an example of the second supply line in the present invention.

The chromatography paper 29b is placed on a placement table 34b. The chromatography paper 29b is disposed between the amplification member 50x and the second button 15b in the vertical direction. The chromatography paper 29b includes the one end portion. The chromatography paper 29b is an example of the test strip in the present invention. Details of the placement table 34b will be described later.

The one end portion is an end portion of the chromatography paper 29b on the upstream side. The one end portion is disposed further upstream than an upstream end portion (not illustrated) of the placement table 34b. In other words, the one end portion protrudes from the upstream end portion of the placement table 34b to the upstream side.

The placement table 34b is a table on which the chromatography paper 29b is placed.

The following description is an example of a case in which the amplified nucleic acid is supplied to the chromatography paper 29b as a result of the second button 15b being operated.

First, the second button 15b is operated by a user.

Then, the second button 15b presses the absorbent member 19b attached to the one end portion of the chromatography paper 29b toward the amplification member 50x.

Then, the absorbent member 19b is connected to the amplification member 50x. That is, the absorbent member 19b connects the amplification member 50x to the chromatography paper 29b. In other words, when the second button 15b is operated, the amplification member 50x is connected to the chromatography paper 29b via the absorbent member 19b.

Then, the detection solution generated in the amplification member 50x is supplied to the chromatography paper 29b through the absorbent member 19b. In other words, the amplified nucleic acid is supplied to the chromatography paper 29b through the absorbent member 19b.

In this way, when the second button 15b is operated, the amplified nucleic acid is supplied to the chromatography paper 29b. In other words, the second button 15b causes the amplified nucleic acid to be supplied to the chromatography paper 29b.

### Fourth Embodiment of Present Apparatus and Present Method

The fourth embodiment of the present apparatus and the present method will be described below.

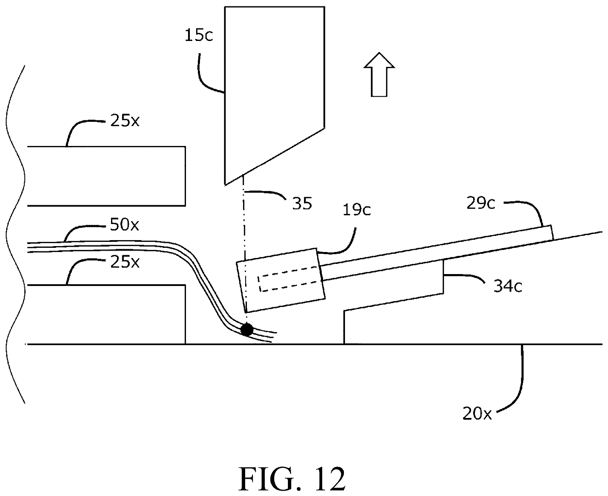

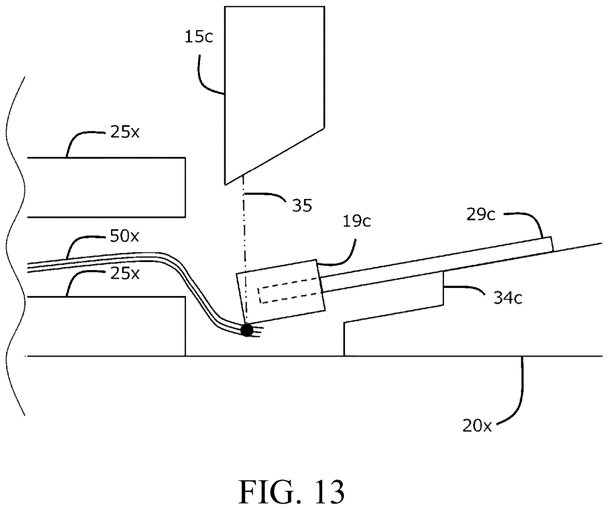

Fig. 12 is a schematic diagram illustrating yet another embodiment of the present apparatus and illustrating a state in which a second button 15c is being operated. In the figure, a white arrow indicates a moving direction of the second button 15c when the second button 15c is operated (i.e., when the second button 15c is raised by a user). Fig. 13 is a schematic diagram illustrating a state in which the amplification member 50x is connected to a chromatography paper 29c.

The second button 15c is a button for connecting the amplification member 50x to an absorbent member 19c. The second button 15c is disposed above the amplification member 50x. The second button 15c is disposed above the absorbent member 19c. The second button 15c is an example of the supply operating member in the present invention.

The absorbent member 19c supplies the detection solution generated in the amplification member 50x in the tube 25x to the chromatography paper 29c. The absorbent member 19c is attached to one end portion (not illustrated) of the chromatography paper 29c on the upstream side in the supply direction of the amplified nucleic acid. The absorbent member 19c is disposed between the amplification member 50x and the second button 15c in the vertical direction. The absorbent member 19c is an example of the second supply line in the present invention. Details of the one end portion will be described later.

The chromatography paper 29c is placed on a placement table 34c. The chromatography paper 29c is disposed between the amplification member 50x and the second button 15c in the vertical direction. The chromatography paper 29c includes the one end portion. The chromatography paper 29c is an example of the test strip in the present invention.

The one end portion is an end portion of the chromatography paper 29c on the upstream side. The one end portion is disposed further upstream than an upstream end portion (not illustrated) of the placement table 34c. In other words, the one end portion protrudes from the upstream end portion of the placement table 34c to the upstream side.

The placement table 34c is a table on which the chromatography paper 29c is placed.

A connecting member 35 is a member that connects the amplification member 50x to the second button 15c. The connecting member 35 has a thread-like shape.

Herein, the second button 15c causes the amplification member 50x to be connected to the absorbent member 19c. That is, when the second button 15c is operated, the amplification member 50x connected to the connecting member 35 is connected to the absorbent member 19c.

The following description is an example of a case in which the amplified nucleic acid is supplied to the chromatography paper 29c as a result of the second button 15c being operated.

First, the second button 15c is operated by a user.

Then, the amplification member 50x connected to the connecting member 35 is connected to the absorbent member 19c. That is, the absorbent member 19c connects the amplification member 50x to the chromatography paper 29c. In other words, when the second button 15c is operated, the amplification member 50x is connected to the chromatography paper 29c via the absorbent member 19c.

Then, the detection solution generated in the amplification member 50x is supplied to the chromatography paper 29c through the absorbent member 19c. In other words, the amplified nucleic acid is supplied to the chromatography paper 29c through the absorbent member 19c.

In this way, when the second button 15c is operated, the amplified nucleic acid is supplied to the chromatography paper 29c. In other words, the second button 15c causes the amplified nucleic acid to be supplied to the chromatography paper 29c.

### Fifth Embodiment of Present Apparatus and Present Method

The fifth embodiment of the present apparatus and the present method will be described below.

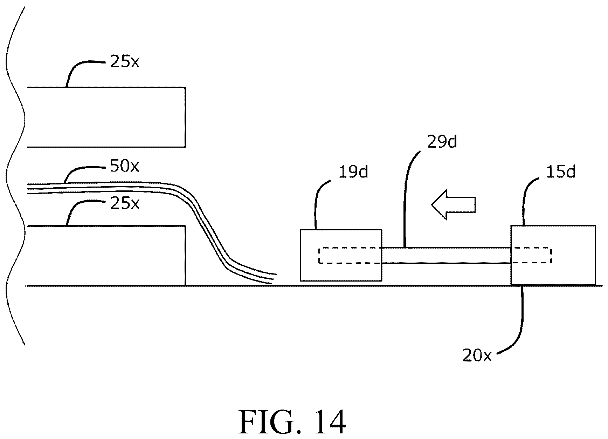

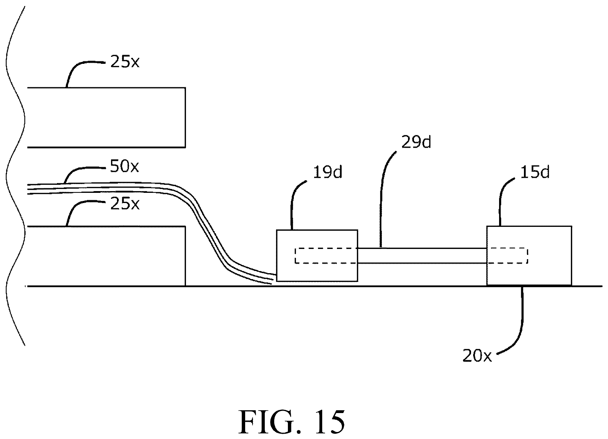

Fig. 14 is a schematic diagram illustrating yet another embodiment of the present apparatus and illustrating a state in which a second button 15d is being operated. In the figure, a white arrow indicates a moving direction of the second button 15d when the second button 15d is operated (i.e., when the second button 15d is pressed by a user). Fig. 15 is a schematic diagram illustrating a state in which the amplification member 50x is connected to a chromatography paper 29d.

The second button 15d is a button for pressing an absorbent member 19d. The second button 15d is attached to the other end portion (not illustrated) of the chromatography paper 29d on the downstream side. That is, the second button 15d is disposed on the downstream side with respect to the chromatography paper 29d in the supply direction of the amplified nucleic acid. The second button 15d is an example of the supply operating member in the present invention. Details of the other end portion will be described later.

The absorbent member 19d supplies the detection solution generated in the amplification member 50x in the tube 25x to the chromatography paper 29d. The absorbent member 19d is attached one end portion (not illustrated) of the chromatography paper 29d on the upstream side in the supply direction of the amplified nucleic acid. The absorbent member 19d is disposed between the amplification member 50x and the second button 15d in the supply direction of the amplified nucleic acid. Details of the one end portion will be described later.

Herein, the second button 15d presses the absorbent member 19d. That is, when the second button 15d is operated, the absorbent member 19d is pressed toward the amplification member 50x by the second button 15d. As a result, the absorbent member 19d is connected to the amplification member 50x. The absorbent member 19d is an example of the second supply line in the present invention.

The chromatography paper 29d is disposed between the amplification member 50x and the second button 15d in the supply direction of the amplified nucleic acid. The chromatography paper 29d includes the one end portion and the other end portion. The chromatography paper 29d is an example of the test strip in the present invention.

The one end portion is an end portion of the chromatography paper 29d on the upstream side.

The other end portion is an end portion of the chromatography paper 29d on the downstream side.

The following description is an example of a case in which the amplified nucleic acid is supplied to the chromatography paper 29d as a result of the second button 15d being operated.

First, the second button 15d is operated by a user.

Then, the second button 15d presses the absorbent member 19d attached to the one end portion of the chromatography paper 29d.

Then, the absorbent member 19d is connected to the amplification member 50x. That is, the absorbent member 19d connects the amplification member 50x to the chromatography paper 29d. In other words, when the second button 15d is operated, the amplification member 50x is connected to the chromatography paper 29d via the absorbent member 19d.

Then, the detection solution generated in the amplification member 50x is supplied to the chromatography paper 29d through the absorbent member 19d. In other words, the amplified nucleic acid is supplied to the chromatography paper 29d through the absorbent member 19d.

In this way, when the second button 15d is operated, the amplified nucleic acid is supplied to the chromatography paper 29d. In other words, the second button 15d causes the amplified nucleic acid to be supplied to the chromatography paper 29d.

### Configuration of Heating Apparatus

The present heating apparatus heats the present apparatus. The present apparatus is the detection apparatus according to the first to fifth embodiments. That is, the present apparatus heated by the present heating apparatus detects an amplified nucleic acid generated from a target nucleic acid contained in a specimen.

The following description is an example when the present heating apparatus heats the present apparatus 1a according to the second embodiment.

In the following description, the specimen (saliva) containing the target nucleic acid is introduced in advance into the present apparatus 1a. That is, the introduced saliva is absorbed into the tube 25a. That is, the saliva is supplied to the tube 25a.

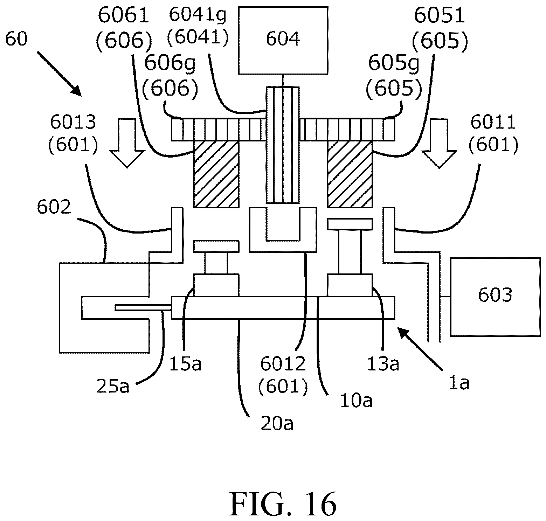

Fig. 16 is a schematic diagram illustrating an embodiment of the present heating apparatus. The figure illustrates that the present apparatus 1a is accommodated in the present heating apparatus 60. In the figure, white arrows indicate the moving directions of a first button pressing portion 605 and a second button pressing portion 606.

The present heating apparatus 60 is an apparatus that heats the present apparatus 1a. The present heating apparatus 60 includes a heating housing 601, a heating unit 602, a heating start button 603, a motor 604, a first button pressing portion 605, a second button pressing portion 606, and a controller (not illustrated).

The heating housing 601 accommodates the present apparatus 1a. The heating housing 601 includes a first housing 6011, a second housing 6012, and a third housing 6013.

The first housing 6011 constitutes the heating housing 601 together with the second housing 6012 and the third housing 6013.

The second housing 6012 constitutes the heating housing 601 together with the first housing 6011 and the third housing 6013. A first hole (not illustrated) through which the first button pressing portion 605 is inserted is formed between the first housing 6011 and the second housing 6012.

The third housing 6013 constitutes the heating housing 601 together with the first housing 6011 and the second housing 6012. A second hole (not illustrated) through which the second button pressing portion 606 is inserted is formed between the second housing 6012 and the third housing 6013.

The heating unit 602 heats the tube 25a.

Herein, the saliva contains the target nucleic acid. The saliva is supplied to the tube 25a. That is, the heating unit 602 heats the target nucleic acid.

The heating start button 603 is a button for starting the heating of the present apparatus 1a. The heating start button 603 is an example of a heating start member in the present invention.

The motor 604 is a power source for supplying rotational power. The motor 604 supplies (transmits) the rotational power to the first button pressing portion 605 and the second button pressing portion 606. The motor 604 includes a motor rotary shaft 6041. The transmission of the rotational power will be described later.

The motor rotary shaft 6041 rotates by the rotational power from the motor 604. The motor rotary shaft 6041 is disposed between the first button pressing portion 605 and the second button pressing portion 606. The motor rotary shaft 6041 includes a gear portion 6041g.

The gear portion 6041g is a gear composed of a plurality of teeth (not illustrated) and a plurality of grooves (not illustrated). The gear portion 6041g meshes with a gear portion 605g and a gear portion 606g. Details of the gear portion 605g and the gear portion 606g will be described later.

The first button pressing portion 605 is a member that presses the first button 13a. The first button pressing portion 605 is disposed above the first hole. The first button pressing portion 605 includes a first pressing portion 6051 and the gear portion 605g.

The first pressing portion 6051 presses the first button 13a. The first pressing portion 6051 is disposed below the gear portion 605g. The first pressing portion 6051 is connected to the gear portion 605g.

The gear portion 605g is a gear composed of a plurality of teeth and a plurality of grooves. The gear portion 605g meshes with the gear portion 6041g.

The second button pressing portion 606 is a member that presses the second button 15a. The second button pressing portion 606 is disposed above the second hole. The second button pressing portion 606 includes a second pressing portion 6061 and the gear portion 606g.

The second pressing portion 6061 presses the second button 15a. The second pressing portion 6061 is disposed below the gear portion 606g. The second pressing portion 6061 is connected to the gear portion 606g.

The gear portion 606g is a gear composed of a plurality of teeth and a plurality of grooves. The gear portion 606g meshes with the gear portion 6041g.

The control unit controls the operation of the heating unit 602 and the operation of the motor 604. The operation of the heating unit 602 includes starting the heating of the present apparatus 1a and stopping the heating of the present apparatus 1a. The operation of the motor 604 includes starting the rotation of the motor rotary shaft 6041 and stopping the rotation of the motor rotary shaft 6041.

The control unit notifies that a predetermined time has elapsed (the operation is completed) from when the heating start button 603 is pressed.

An aspect of the notification by the control unit is, for example, display of the completion of the operation on a display (not illustrated) of the present heating apparatus 60, output of a sound from a sound source output device (not illustrated) of the present heating apparatus 60, or lighting of an indicator lamp (not illustrated) of the present heating apparatus 60.

### Transmission of Rotational Power

In the following description, the first button 13a is disposed in the first hole. The second button 15a is disposed in the second hole.

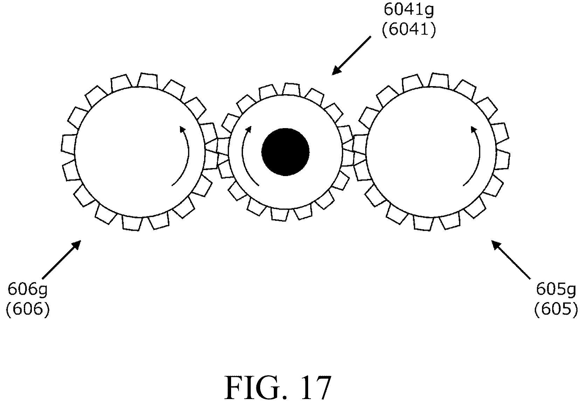

Fig. 17 is a schematic diagram illustrating a state, as viewed from the motor 604 side, in which the first button pressing portion 605, the second button pressing portion 606, and the motor rotary shaft 6041 are rotating. In the figure, a black circle indicates a central axis (not illustrated) of the motor rotary shaft 6041. In the figure, a clockwise arrow indicates the direction of rotation of the motor rotary shaft 6041. The counterclockwise arrows indicate the directions of rotation of the first button pressing portion 605 and the second button pressing portion 606.

The motor rotary shaft 6041 rotates in the clockwise direction by the rotational power from the motor 604. That is, the gear portion 6041g rotates in the clockwise direction.

Herein, the gear portion 6041g is disposed between the gear portion 605g and the gear portion 606g. The gear portion 6041g meshes with the gear portion 605g and the gear portion 606g. That is, when the gear portion 6041g rotates clockwise, the gear portion 605g and the gear portion 606g rotate counterclockwise. That is, the rotational power from the motor 604 is transmitted to the gear portion 605g and the gear portion 606g. In other words, the rotational power from the motor 604 is transmitted to the first button pressing portion 605 and the second button pressing portion 606 via the motor rotary shaft 6041.

When the gear portion 605g rotates counterclockwise, the first button pressing portion 605 moves toward the first hole. As a result, the first pressing portion 6051 presses the first button 13a. When the gear portion 606g rotates counterclockwise, the second button pressing portion 606 moves toward the second hole. As a result, the second pressing portion 6061 presses the second button 15a.

### Operation of Heating Apparatus

The following description is an example of a case in which the present heating apparatus 60 heats the present apparatus 1a and presses the buttons (i.e., the first button 13a and the second button 15a) of the present apparatus 1a.

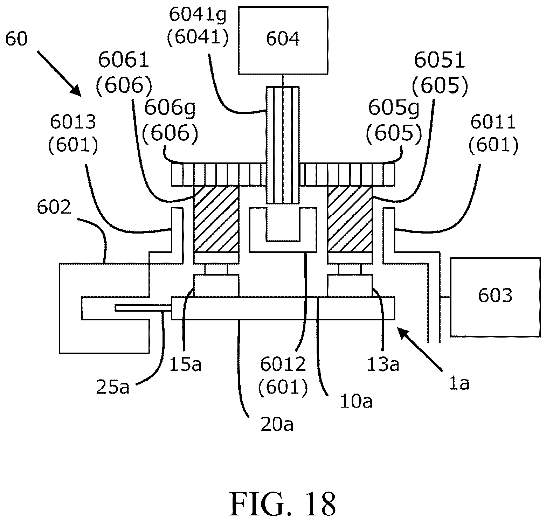

Fig. 18 is a schematic diagram illustrating a state in which the first button 13a and the second button 15a are pressed by the first button pressing portion 605 and the second button pressing portion 606, respectively.

First, the present apparatus 1a is accommodated in the heating housing 601.

Then, when the heating start button 603 is operated, the control unit controls the operation of the heating unit 602. That is, when the heating start button 603 is operated, the heating unit 602 starts heating the present apparatus 1a. More specifically, when the heating start button 603 is operated, the heating of the target nucleic acid in the present apparatus 1a is started. In other words, the heating start button 603 starts the heating of the target nucleic acid by the heating unit 602.

Then, the control unit controls the operation of the heating unit 602 when a predetermined time (e.g., 30 minutes) has elapsed from the start of the heating of the present apparatus 1a. That is, when the predetermined time has elapsed from the start of the heating of the present apparatus 1a, the heating unit 602 stops the heating of the present apparatus 1a. In other words, when the heating start button 603 is operated, the heating unit 602 heats the target nucleic acid for the predetermined time.

Then, the control unit controls the operation of the motor 604. That is, the rotation of the motor rotary shaft 6041 is started. More specifically, the motor rotary shaft 6041 rotates together with the first button pressing portion 605 and the second button pressing portion 606. As a result, the first button pressing portion 605 moves toward the first hole. The second button pressing portion 606 moves toward the second hole (see Figs. 16 and 17).

Then, the control unit controls the operation of the motor 604 when a predetermined time (e.g., three seconds) has elapsed from the start of the rotation of the motor rotary shaft 6041. That is, when the predetermined time has elapsed from the start of the rotation of the motor rotary shaft 6041, the rotation of the motor rotary shaft 6041 is stopped. In this state, the first button 13a is pressed by the first pressing portion 6051. The second button 15a is pressed by the second press portion 6061 (see Fig. 18).

Herein, the distance between the first button 13a and the first pressing portion 6051 is shorter than the distance between the second button 15a and the second pressing portion 6061. That is, the first button 13a is pressed by the present heating apparatus 60 prior to the second button 15a.

Note that, in the present invention, the distance between the first button and the first pressing portion may be the same as the distance between the second button and the second pressing portion.

Then, the control unit notifies that a predetermined time (e.g., 35 minutes) has elapsed since the pressing of the heating start button 603.

### Conclusion (3)

As described above, the present heating apparatus 60 includes the heating housing 601, the heating unit 602, and the heating start button 603. The heating housing 601 accommodates the present apparatus 1a. The heating start button 603 causes the heating unit 602 to heat the present apparatus 1a accommodated in the heating housing 601. When the heating start button 603 is operated, the heating unit 602 heats the present apparatus 1a accommodated in the heating housing 601 for a predetermined time. In other words, when the heating start button 603 is operated, the target nucleic acid in the present apparatus 1a is heated by the heating unit 602 for the predetermined time. That is, the target nucleic acid is heated for the predetermined time solely by the operation of the heating start button 603 by a user. Accordingly, the present heating apparatus 60 is capable of performing heating of the target nucleic acid without relying on the user's expertise or skill.

As described above, the present heating apparatus 60 includes the motor 604, the first button pressing portion 605, the second button pressing portion 606, and the control unit. The motor 604 supplies (transmits) the rotational power to the first button pressing portion 605 and the second button pressing portion 606. The first button pressing portion 605 presses the first button 13a, based on the rotational power from the motor 604. The second button pressing portion 606 presses the second button 15a, based on the rotational power from the motor 604. The control unit controls the operation of the heating unit 602 and the operation of the motor 604 at a predetermined timing. The operation of the heating unit 602 includes starting the heating of the present apparatus 1a and stopping the heating of the present apparatus 1a. The operation of the motor 604 includes starting the rotation of the motor rotary shaft 6041 and stopping the rotation of the motor rotary shaft 6041. The predetermined timing is, for example, when the heating start button 603 is operated, when a predetermined time (e.g., 30 minutes) has elapsed from the start of heating of the present apparatus 1a, or when a predetermined time (e.g., 3 seconds) has elapsed from the start of rotation of the motor rotary shaft 6041. That is, the control unit controls the operation of the heating unit 602 and the operation of the motor 604 solely by the operation of the heating start button 603 by a user. In other words, the present heating apparatus 60 is capable of performing heating of the apparatus 1a and pressing of the buttons (i.e., the first button 13a and the second button 15a) without relying on the user's expertise or skill.

### Conclusion

In the embodiments described above, the present apparatus (1, 1a) is used for determining whether a subject (human) is infected with a specific pathogen. However, the present apparatus may be used for purposes other than determining whether a human is infected with a specific pathogen. That is, for example, the present apparatus may be used for determining whether an animal is infected with a specific pathogen. The present apparatus may be used for identification of various biological species. Furthermore, the present apparatus may be used for determination of genetic abnormalities in humans and animals. Furthermore, the present apparatus may be used for detecting a single nucleotide mutation in drug-resistant bacteria.

In the embodiments described above, the target nucleic acid is a nucleic acid derived from a microorganism that may be contained in saliva of a subject (human). However, the target nucleic acid in the present invention is not limited to nucleic acids derived from microorganisms that may be contained in human saliva. That is, for example, the target nucleic acid in the present invention may be a nucleic acid derived from a microorganism that may be contained in a specimen from an animal or a nucleic acid derived from a non-pathogenic microorganism.

In the embodiments described above, the specimen is saliva of a subject (human). However, the specimen in the present invention is not limited to human saliva. That is, for example, the specimen in the present invention may be sputum, blood, serum, plasma, otorrhea, urine, or a nasopharyngeal swab from a human.

In the embodiments described above, the heating means is the heater (30, 30a) including the upper heater (31, 31a) and the lower heater (32, 32a). That is, the first supply line is heated from above and below. However, in the present invention, the first supply line may be heated, for example, only from above or only from below. The heating means in the present invention is not limited to the heater. That is, the heating means in the present invention may be appropriately selected from means capable of heating the target nucleic acid to a temperature at which the target nucleic acid is amplified, and may be, for example, means using a thermostatic water tank or means utilizing heat generation by a chemical reaction.

In the embodiments described above, the color developing agent is disposed on chromatography paper (29, 29a). However, the location where the color developing agent is disposed in the present invention is not limited to the chromatography paper. That is, for example, the color developing agent in the present invention may be disposed in the developing solution or in the second supply line.

In the embodiments described above, the first supply line is a tube (25 to 28, 25a). However, the first supply line in the present invention is not limited to a tube. That is, for example, the first supply line in the present invention may be constituted by a groove formed in the base.

In the embodiments described above, the method for amplifying a target nucleic acid is the LAMP method that is an isothermal nucleic acid amplification method. However, the method for amplifying a target nucleic acid in the present invention is not limited to the LAMP method. That is, for example, the method for amplifying a target nucleic acid in the present invention may be a strand displacement amplification (SDA) method, a helicase-dependent amplification (HDA) method, a recombinase polymerase amplification (RPA) method, a rolling circle amplification (RCA) method, or a nucleic acid sequence-based amplification (NASBA) method.

In the embodiments described above, the support is a fibrous aggregate. However, the support in the present invention is not limited to a fibrous aggregate. That is, for example, the support in the present invention may be a solid or a polymer having a microstructure, or a solid or a polymer that gels upon water absorption.

In the embodiments described above, the discharge mechanism includes the rupturing member (the needle 23, 23a). That is, the developing solution is discharged from the developing solution tank by the rupturing member rupturing a portion of the developing solution tank (18, 18a). However, the discharge mechanism in the present invention is not limited to the rupturing member rupturing a portion of the developing solution tank. That is, for example, the discharge mechanism in the present invention may be a discharge mechanism having an outflow port of the developing solution tank that slides to discharge the developing solution, or a discharge mechanism having a film disposed at the outflow port, the film exhibiting increased permeability to the developing solution upon heating.

In the embodiments described above, the developing solution is a mixed solution of water, a phosphate buffer, sodium chloride, a surfactant such as Tween20, and bovine serum albumin. However, the developing solution in the present invention is not limited to the mixed solution. That is, for example, the developing solution in the present invention may be appropriately selected from developing solutions used in chromatography.

In the embodiments described above, the developing solution is stored in the developing solution tank (18, 18a). The developing solution is discharged (introduced) downstream from the developing solution tank as a portion of the developing solution tank is ruptured by the needle (23, 23a). However, the discharge mechanism for discharging the developing solution in the downstream direction (i.e., the means for introducing the developing solution into the present apparatus) in the present invention is not limited to the rupture of the developing solution tank. That is, for example, the developing solution may be introduced from a syringe (not illustrated) into the present apparatus. The syringe stores the developing solution. The syringe that stores the developing solution is inserted into the specimen introduction port. The developing solution is introduced into the present apparatus through the specimen introduction port when a pressing member (not illustrated) of the syringe inserted into the specimen introduction port is pressed by a user.

Note that the means for introducing the developing solution stored in the syringe into the present apparatus is not limited to the pressing operation by a user. That is, for example, the developing solution stored in the syringe may be introduced into the present apparatus by a discharge pressure from a pump (not illustrated).

### Features of Present Apparatus, Present Method, and Present Heating Apparatus

The features of the present apparatus, the present method, and the present heating apparatus described above are collectively set forth below.

### Features of Present Apparatus

The present apparatus is a detection apparatus (e.g., present apparatus 1, 1a) that detects an amplified nucleic acid generated from a target nucleic acid contained in a specimen and the detection apparatus includes a specimen placement member (e.g., specimen receiver 22, 22a) in which the specimen is placed, a test strip (e.g., chromatography paper 29, 29a, 29b, 29c, 29d) used for detecting the amplified nucleic acid, an amplification member (e.g., amplification members 50 to 53, 50a) on which a reagent for use in generating the amplified nucleic acid from the target nucleic acid is immobilized, and a supply line (e.g., absorbent member 19, 19a, 19b, 19c, 19d, tubes 25 to 28, 25a) that supplies the amplified nucleic acid toward the test strip, in which the amplification member is disposed in the supply line.

The present apparatus may include a supply operating member (e.g., second button 15, 15a, 15b, 15c, 15d) that causes the amplified nucleic acid to be supplied to the test strip, in which the amplified nucleic acid may be supplied to the test strip when the supply operating member is operated.

In the present apparatus, the amplification member may be connected to the test strip when the supply operating member is operated.

In the present apparatus, the test strip may be disposed between the amplification member and the supply operating member.

In the present apparatus, the supply operating member may be disposed above the amplification member.

In the present apparatus, the supply operating member may be disposed on a downstream side relative to the test strip in a supply direction of the amplified nucleic acid.

In the present apparatus, the supply line may include a first supply line (e.g., tubes 25 to 28, 25a) connected to the specimen placement member and a second supply line (e.g., absorbent member 19, 19a, 19b, 19c, 19d) that connects the amplification member to the test strip, the amplification member may be disposed in the first supply line, and the amplification member may be connected to the test strip via the second supply line when the supply operating member is operated.

In the present apparatus, the second supply line may be disposed between the first supply line and the test strip when the supply operating member is operated.

In the present apparatus, the second supply line may be attached to one end portion of the test strip on an upstream side (e.g., one end portion of chromatography paper 29b, 29c, or 29d on an upstream side) in the supply direction of the amplified nucleic acid.

In the present apparatus, the second supply line may be disposed between the amplification member and the supply operating member.

In the present apparatus, the supply operating member may be disposed above the amplification member, and the second supply line may be pressed by the supply operating member toward the amplification member when the supply operating member is operated.

The present apparatus may include a connecting member (e.g., connecting member 35) that connects the amplification member to the supply operating member, in which the amplification member connected to the connecting member may be connected to the second supply line when the supply operating member is operated.

The present apparatus may include a placement table (e.g., placement table 34b, 34c) on which the test strip is placed, in which the one end portion may be disposed further upstream than an upstream end portion of the placement table (e.g., upstream end portion of the placement table 34b or 34c).

In the present apparatus, the supply operating member may be disposed on the downstream side relative to the test strip in the supply direction of the amplified nucleic acid, and the second supply line may be pressed by the supply operating member toward the amplification member when the supply operating member is operated.

In the present apparatus, the supply operating member may be attached to the other end portion of the test strip on the downstream side (e.g., the other end portion of the chromatography paper 29d on the downstream side).

The present apparatus may include a housing (e.g., cover 10, 10a, base 20, 20a) that accommodates the specimen placement member, the test strip, the supply line, and the amplification member, in which the housing may include an insertion hole (e.g., upper heater insertion hole 11, lower heater insertion hole 21) into which heating means (e.g., heater 30, upper heater 31, lower heater 32) for heating the first supply line is inserted.

The present apparatus may include a housing that accommodates the specimen placement member, the test strip, and the amplification member, in which a portion of the first supply line may be accommodated in the housing, and the remaining portion of the first supply line may be exposed outside the housing so as to be heated by heating means.

In the present apparatus, the housing may include a specimen introduction port (e.g., specimen introduction port 17, 17a) into which the specimen to be placed in the specimen placement member is introduced, and a sealing member (e.g., cap 12, 12a) that seals the specimen introduction port, in which the inside of the housing may communicate with the outside of the housing only through the specimen introduction port when the specimen introduction port is not sealed by the sealing member.

In the present apparatus, the housing may include an observation window (e.g., second window 16) that allows the test strip to be visible from outside the housing.

In the present apparatus, the test strip may include a color developing surface that develops color when the amplified nucleic acid is captured, and the observation window may be disposed in the housing in such a way as to face the color development surface.

The present apparatus may include a developing solution tank (e.g., developing solution tank 18, 18a) in which a developing solution for supplying the amplified nucleic acid toward the test strip is stored, and a discharge mechanism (e.g., first button 13, 13a, needle 23, 23a) that discharges the developing solution from the developing solution tank, in which the developing solution may be supplied to the supply line when the discharge mechanism is operated.

In the present apparatus, the discharge mechanism may include a movement operating member (e.g., first button 13) that causes the developing solution tank to move, and a rupturing member (e.g., needle 23, 23a) that ruptures a portion of the developing solution tank, when the movement operating member is operated and the developing solution tank is moved.

In the present apparatus, the first supply line may be a tube body, and the amplification member may be disposed inside the tube body.

In the present apparatus, the amplification member may be a linear body, and the tube body may include a first opening (e.g., first openings 251 to 281) disposed on a side of the specimen placement member and a second opening (e.g., second openings 252 to 282) disposed on a side of the test strip, in which one end (e.g., one ends 501 to 531, 501a) of the amplification member may be disposed on a side of the first opening, and the other end (e.g., the other ends 502 to 532, 502a) of the amplification member may be disposed on a side of the second opening.

In the present apparatus, the one end of the amplification member may be exposed outside the tube body from the first opening, and the other end of the amplification member may be exposed outside the tube body from the second opening.

In the present apparatus, the amplification member may include a support that carries the reagent.

In the present apparatus, the support may be a fibrous aggregate.

In the present apparatus, the reagent may include a first reagent that causes the nucleic acid to be amplified and a second reagent that causes the nucleic acid to be amplified, and the linear body may include a first linear body disposed inside the tube body and a second linear body disposed inside the tube body, in which the first reagent is immobilized on the first linear body, and the second reagent is immobilized on the second linear body.

In the present apparatus, the tube body may include a first tube body in which the first linear body is disposed, and a second tube body in which the second linear body is disposed.

In the present apparatus, the target nucleic acid may include a first nucleic acid and a second nucleic acid, in which the first reagent may cause the first nucleic acid to be amplified, and the second reagent may cause the second nucleic acid to be amplified.

The present apparatus may include a color developing agent that binds to the amplified nucleic acid, in which the color developing agent may include a first color developing agent that binds to a first amplified nucleic acid generated by amplifying the first nucleic acid, and a second color developing agent that binds to a second amplified nucleic acid generated by amplifying the second nucleic acid.

The present apparatus may include a capturing agent that captures the amplified nucleic acid bound to the color developing agent, in which the capturing agent may include a first capturing agent that captures the amplified nucleic acid bound to the first color developing agent, and a second capturing agent that captures the amplified nucleic acid bound to the second color developing agent.

In the present apparatus, the capturing agent may be disposed on the test strip, and a position of the first capturing agent on the test strip may be different from a position of the second capturing agent on the test strip.

In the present apparatus, at least one of a developing solution that supplies the amplified nucleic acid toward the test strip, the supply line, or the test strip may include the color developing agent.

In the present apparatus, a detection solution containing the amplified nucleic acid generated by contact between the specimen and the reagent may be supplied to the second supply line, the second supply line may include a first abutment portion (e.g., first abutment portion 191, 191a) that abuts the amplification member and a second abutment portion (e.g., second abutment portion 192, 192a) that abuts the test strip, and the detection solution may be supplied from the first abutment portion to the second supply line and from the second abutment portion to the test strip.

In the present apparatus, the second supply line may be an absorbent member (e.g., absorbent member 19, 19a) capable of absorbing the detection solution, and the detection solution absorbed into the absorbent member may be supplied to the test strip when the supply operating member is operated and the absorbent member is placed between the first supply line and the test strip.

### Features of Present Method

The present method is a method executed by a detection apparatus (e.g., present apparatus 1, 1a) that detects an amplified nucleic acid generated from a target nucleic acid contained in a specimen, the detection apparatus including a specimen placement member (e.g., specimen receiver 22, 22a) in which the specimen is placed, a test strip (e.g., chromatography paper 29, 29a) used for detecting the amplified nucleic acid, an amplification member (e.g., amplification members 50 to 53, 50a) on which a reagent for use in generating the amplified nucleic acid from the target nucleic acid is immobilized, and a supply line (e.g., absorbent member 19, 19a, tubes 25 to 28, 25a) that supplies the amplified nucleic acid toward the test strip, in which the amplification member is disposed in the supply line, the method including a placing step of placing the specimen in the specimen placement member, a generating step of generating the amplified nucleic acid in the supply line, and a detecting step of detecting the amplified nucleic acid on the test strip.

In the present method, the generating step may include a heating step of heating the specimen.

In the present method, the detecting step may include a supplying step of supplying the detection solution containing the amplified nucleic acid to the test strip via the supply line.

### Features of Heating Apparatus

The present heating apparatus includes a heating housing (e.g., heating housing 601) that accommodates a detection apparatus that detects an amplified nucleic acid generated from a target nucleic acid contained in a specimen, a heating unit (e.g., heating unit 602) that heats the target nucleic acid, and a heating start member (e.g., heating start button 603) that causes the heating unit to heat the target nucleic acid, in which the heating unit heats the target nucleic acid for a predetermined time when the heating start member is operated.

In the present heating apparatus, the detection apparatus may be the present apparatus (e.g., present apparatus 1, 1a).

### [Reference Signs List]

1 Detection apparatus
10 Cover (Housing)
11 Upper heater insertion hole
12 Cap (Sealing member)
13 First button (Movement operating member)
14 First window (Amplification observation window)
15 Second button (Supply operating member)
16 Second window (Detection observation window)
17 Specimen introduction port
18 Developing solution tank
19 Absorbent member (Second supply line)
191 First abutment portion
192 Second abutment portion
20 Base (Housing)
21 Lower heater insertion hole
22 Specimen receiver (Specimen placement member)
23 Needle (Rupturing member)
24 Tube mounting member
25 Soft tube (First supply line)
26 Soft tube (First supply line)
27 Soft tube (First supply line)
28 Soft tube (First supply line)
251 First opening
261 First opening
271 First opening
281 First opening
252 Second opening
262 Second opening
272 Second opening
282 Second opening
29 Chromatography paper (Test strip)
30 Heater
31 Upper heater
32 Lower heater
33 Power connector
40 Power supply
50 Amplification member
51 Amplification member
52 Amplification member
53 Amplification member
501 One end
511 One end
521 One end
531 One end
502 Other end
512 Other end
522 Other end
532 Other end
1a Detection Apparatus
10a Cover (Housing)
12a Cap (Sealing member)
13a First button (Movement operating member)
15a Second button (Supply operating member)
17a Specimen introduction port
18a Developing solution tank
19a Absorbent member (Second supply line)
191a First abutment portion
192a Second abutment portion
20a Base (Housing)
22a Specimen receiver (Specimen placement member)
23a Needle (Rupturing member)
25a Soft tube (First supply line)
29a Chromatography paper (Test strip)
30a Heater
31a Upper heater
32a Lower heater
50a Amplification member
501a One end
502a Other end
15b Second button (Supply operating member)
19b Absorbent member (Second supply line)
29b Chromatography paper (Test strip)
34b Placement table
15c Second button (Supply operating member)
19c Absorbent member (Second supply line)
29c Chromatography paper (Test strip)
34c Placement table
35 Connecting member
15d Second button (Supply operating member)
19d Absorbent member (Second supply line)
29d Chromatography paper (Test strip)
60 Heating apparatus
601 Heating housing
6011 First housing
6012 Second housing
6013 Third housing
602 Heating unit
603 Heating start button (Heating start member)
604 Motor
6041 Motor rotary shaft
6041g Gear portion
605 First button pressing portion
6051 First pressing portion
605g Gear portion
606 Second button pressing portion
6061 Second pressing portion
606g Gear portion

## Claims

1. A detection apparatus for detecting an amplified nucleic acid generated from a target nucleic acid contained in a specimen, the detection apparatus comprising:
a specimen placement member in which the specimen is placed;
a test strip used for detecting the amplified nucleic acid;
an amplification member on which a reagent for use in generating the amplified nucleic acid from the target nucleic acid is immobilized; and
a supply line configured to supply the amplified nucleic acid toward the test strip, wherein
the amplification member is disposed in the supply line.

2. The detection apparatus according to claim 1, further comprising a supply operating member configured to supply the amplified nucleic acid to the test strip, wherein the amplified nucleic acid is supplied to the test strip when the supply operating member is operated.

3. The detection apparatus according to claim 2, wherein the amplification member is connected to the test strip when the supply operating member is operated.

4. The detection apparatus according to claim 3, wherein the test strip is disposed between the amplification member and the supply operating member.

5. The detection apparatus according to claim 4, wherein the supply operating member is disposed above the amplification member.

6. The detection apparatus according to claim 4, wherein the supply operating member is disposed on a downstream side relative to the test strip in a supply direction of the amplified nucleic acid.

7. The detection apparatus according to claim 3, wherein
the supply line includes
a first supply line connected to the specimen placement member, and
a second supply line that connects the amplification member to the test strip,
the amplification member is disposed in the first supply line, and
the amplification member is connected to the test strip via the second supply line when the supply operating member is operated.

8. The detection apparatus according to claim 7, wherein the second supply line is disposed between the first supply line and the test strip when the supply operating member is operated.

9. The detection apparatus according to claim 7, wherein the second supply line is attached to one end portion of the test strip on an upstream side in a supply direction of the amplified nucleic acid.

10. The detection apparatus according to claim 9, wherein the second supply line is disposed between the amplification member and the supply operating member.

11. The detection apparatus according to claim 10, wherein
the supply operating member is disposed above the amplification member, and
the second supply line is pressed by the supply operating member toward the amplification member when the supply operating member is operated.

12. The detection apparatus according to claim 10, further comprising a connecting member that connects the amplification member to the supply operating member, wherein
the amplification member connected to the connecting member is connected to the second supply line when the supply operating member is operated.

13. The detection apparatus according to claim 11 or 12, further comprising a placement table on which the test strip is placed, wherein
the one end portion is disposed further upstream than an upstream end portion of the placement table.

14. The detection apparatus according to claim 10, wherein
the supply operating member is disposed on a downstream side relative to the test strip in the supply direction of the amplified nucleic acid, and
the second supply line is pressed by the supply operating member toward the amplification member when the supply operating member is operated.

15. The detection apparatus according to claim 14, wherein the supply operating member is attached to the other end portion of the test strip on the downstream side.

16. The detection apparatus according to claim 8, further comprising a housing that accommodates the specimen placement member, the test strip, the supply line, and the amplification member, wherein
the housing includes an insertion hole into which heating means for heating the first supply line is inserted.

17. The detection apparatus according to claim 8, further comprising a housing that accommodates the specimen placement member, the test strip, and the amplification member, wherein
a portion of the first supply line is accommodated in the housing, and
the remaining portion of the first supply line is exposed outside the housing so as to be heated by heating means.

18. The detection apparatus according to claim 16 or 17, wherein
the housing includes
a specimen introduction port into which the specimen to be placed in the specimen placement member is introduced, and
a sealing member configured to seal the specimen introduction port, wherein
the inside of the housing communicates with the outside of the housing only through the specimen introduction port when the specimen introduction port is not sealed by the sealing member.

19. The detection apparatus according to claim 16 or 17, wherein
the housing includes an observation window that allows the test strip to be visible from outside the housing.

20. The detection apparatus according to claim 19, wherein
the test strip includes a color developing surface that develops color when the amplified nucleic acid is captured, and
the observation window is disposed in the housing in such a way as to face the color development surface.

21. The detection apparatus according to claim 1, further comprising:
a developing solution tank in which a developing solution for supplying the amplified nucleic acid toward the test strip is stored; and
a discharge mechanism configured to discharge the developing solution from the developing solution tank, wherein
the developing solution is supplied to the supply line when the discharge mechanism is operated.

22. The detection apparatus according to claim 21, wherein
the discharge mechanism includes
a movement operating member configured to move the developing solution tank, and
a rupturing member configured to rupture a portion of the developing solution tank, when the movement operating member is operated and the developing solution tank is moved.

23. The detection apparatus according to claim 18, wherein the developing solution for supplying the amplified nucleic acid toward the test strip is introduced through the specimen introduction port.

24. The detection apparatus according to claim 8, wherein
the first supply line is a tube body, and
the amplification member is disposed inside the tube body.

25. The detection apparatus according to claim 24, wherein
the amplification member is a linear body, and
the tube body includes
a first opening disposed on a side of the specimen placement member, and
a second opening disposed on a side of the test strip, wherein
one end of the amplification member is disposed on a side of the first opening, and
the other end of the amplification member is disposed on a side of the second opening.

26. The detection apparatus according to claim 25, wherein
the one end of the amplification member is exposed outside the tube body from the first opening, and
the other end of the amplification member is exposed outside the tube body from the second opening.

27. The detection apparatus according to claim 26, wherein the amplification member includes a support configured to carry the reagent.

28. The detection apparatus according to claim 27, wherein the support is a fibrous aggregate.

29. The detection apparatus according to claim 25, wherein
the reagent includes
a first reagent configured to amplify the target nucleic acid, and
a second reagent configured to amplify the target nucleic acid, and
the linear body includes
a first linear body disposed inside the tube body, and
a second linear body disposed inside the tube body, wherein
the first reagent is immobilized on the first linear body, and
the second reagent is immobilized on the second linear body.

30. The detection apparatus according to claim 29, wherein the tube body includes
a first tube body in which the first linear body is disposed, and
a second tube body in which the second linear body is disposed.

31. The detection apparatus according to claim 29, wherein
the target nucleic acid includes a first nucleic acid and a second nucleic acid, wherein
the first reagent is configured to amplify the first nucleic acid, and
the second reagent is configured to amplify the second nucleic acid.

32. The detection apparatus according to claim 31, further comprising a color developing agent configured to bind to the amplified nucleic acid, wherein
the color developing agent includes
a first color developing agent configured to bind to a first amplified nucleic acid generated by amplifying the first nucleic acid, and
a second color developing agent configured to bind to a second amplified nucleic acid generated by amplifying the second nucleic acid.

33. The detection apparatus according to claim 32, further comprising a capturing agent configured to capture the amplified nucleic acid bound to the color developing agent, wherein
the capturing agent includes
a first capturing agent configured to capture the amplified nucleic acid bound to the first color developing agent, and
a second capturing agent configured to captures the amplified nucleic acid bound to the second color developing agent.

34. The detection apparatus according to claim 33, wherein
the capturing agent is disposed on the test strip, and
a position of the first capturing agent on the test strip is different from a position of the second capturing agent on the test strip.

35. The detection apparatus according to claim 34, wherein
at least one of a developing solution for supplying the amplified nucleic acid toward the test strip, the supply line, or the test strip includes the color developing agent.

36. The detection apparatus according to claim 8, wherein
a detection solution containing the amplified nucleic acid generated by contact between the specimen and the reagent is supplied to the second supply line,
the second supply line includes
a first abutment portion configured to abut the amplification member, and
a second abutment portion configured to abut the test strip, and
the detection solution is supplied from the first abutment portion to the second supply line and from the second abutment portion to the test strip.

37. The detection apparatus according to claim 36, wherein
the second supply line is an absorbent member capable of absorbing the detection solution, and
the detection solution absorbed into the absorbent member is supplied to the test strip, when the supply operating member is operated and the absorbent member is placed between the first supply line and the test strip.

38. A detection method executed by a detection apparatus configured to detect an amplified nucleic acid generated from a target nucleic acid contained in a specimen, the detection apparatus including a specimen placement member in which the specimen is placed, a test strip used for detecting the amplified nucleic acid, an amplification member on which a reagent for use in generating the amplified nucleic acid from the target nucleic acid is immobilized, and a supply line configured to supply the amplified nucleic acid toward the test strip, wherein the amplification member is disposed in the supply line, the method comprising:
a placing step of placing the specimen in the specimen placement member;
a generating step of generating the amplified nucleic acid in the supply line; and
a detecting step of detecting the amplified nucleic acid on the test strip.

39. The detection method according to claim 38, wherein the generating step includes a heating step of heating the specimen.

40. The detection method according to claim 38, wherein the detecting step includes a supplying step of supplying the detection solution containing the amplified nucleic acid to the test strip via the supply line.

41. A heating apparatus comprising:
a heating housing that accommodates a detection apparatus configured to detect an amplified nucleic acid generated from a target nucleic acid contained in a specimen;
a heating unit configured to heat the target nucleic acid; and
a heating start member configured to heat the target nucleic acid by the heating unit, wherein
the heating unit heats the target nucleic acid for a predetermined time when the heating start member is operated.

42. The heating apparatus according to claim 41, wherein the detection apparatus is the detection apparatus according to claim 1.
